# EUROPEAN PATENT SPECIFICATION

(11) **EP 2 569 328 B1**
(45) Date of publication and mention of the grant of the patent: **14.12.2016**
(21) Application number: 11721899.0
(22) Date of filing: 11.05.2011
(51) Int. Cl.: C12N 15/113, C12N 15/11

(54) **PROGRAMMABLE SELF-ASSEMBLED NANOSTRUCTURES BASED ON SIDECHAIN-MODIFIED PNA FOR THE MULTIVALENT DISPLAY OF LIGANDS**
PROGRAMMIERBARE SELBSTANGEORDNETE NANOSTRUKTUREN AUF DER BASIS VON SEITENKETTENMODIFIZIERTER PNA FÜR POLYVALENTES LIGANDEN-DISPLAY
NANOSTRUCTURES AUTO-ASSEMBLÉES PROGRAMMABLES BASÉES SUR UN APN À CHAÎNES LATÉRALES MODIFIÉES POUR LA PRÉSENTATION MULTIVALENTE DE LIGANDS

(30) Priority: 11.05.2010 US 333442 P
(43) Date of publication of application: 20.03.2013
(73) Proprietor: The U.S.A. as represented by the Secretary, Department of Health and Human Services, Bethesda, MD 20892-7660 (US)
(72) Inventor: APPELLA, Daniel, H., Rockville MD 20850 (US)
(74) Representative: Carpmaels & Ransford LLP
(86) International application number: PCT/US2011/036090
(87) International publication number: WO 2011/143323

(56) References cited:
- WO-A1-2008/039367
- ENGLUND E.A. AND APPELLA D.H.: "[gamma]-substituted Peptide Nucleic Acids constructed from L-Lysine are a versatile scaffold for multifunctional display", ANGEWANDTE CHEMIE INTERNATIONAL EDITION, vol. 46, no. 9, 19 February 2007 (2007-02-19), pages 1414-1418, XP55010852, ISSN: 1433-7851, DOI: 10.1002/anie.200603483 -& Englund E.A. and Appella D.H.: "Supporting Information", , 2007, XP002662760, Retrieved from the Internet: URL:http://www.wiley-vch.de/contents/jc_20 02/2007/z603483_s.pdf [retrieved on 2011-10-31]
- APPELLA D.H.: "Molecular designs for peptide nucleic acids in detection and multivalent platforms", ABSTRACTS OF PAPERS AMERICAN CHEMICAL SOCIETY, vol. 238, August 2009 (2009-08), pages 62-ORGN, XP008144804, & MEETING OF THE DIVISION OF CHEMICAL TOXICOLOGY OF THE AMERICAN-CHEMICAL-SOCIETY HELD AT THE 238TH AC; WASHINGTON, DC, USA; AUGUST 16 -20, 2009 ISSN: 0065-7727
- ENGLUND E.A.: "ORGN 13-Development of gamma-substituted PNA as versatile scaffolds for multivalent display", ABSTRACTS OF PAPERS AMERICAN CHEMICAL SOCIETY, vol. 237, March 2009 (2009-03), pages 13-ORGN, XP008144807, & 237TH NATIONAL MEETING OF THE AMERICAN-CHEMICAL-SOCIETY; SALT LAKE CITY, UT, USA; MARCH 22 -26, 2009 ISSN: 0065-7727
- ENGLUND E.A. AND APPELLA D.H.: "Synthesis of gamma-substituted peptide nucleic acids: a new place to attach fluorophores without affecting DNA binding", ORGANIC LETTERS, AMERICAN CHEMICAL SOCIETY, US, vol. 7, no. 16, 1 August 2005 (2005-08-01) , pages 3465-3467, XP008144826, ISSN: 1523-7060 -& Englund E.A. and Appella D.H.: "Supporting Info", , 1 August 2005 (2005-08-01), XP002662761, Retrieved from the Internet: URL:http://pubs.acs.org/doi/suppl/10.1021/ ol051143z [retrieved on 2011-11-03]
- ZHANG N. AND APPELLA D.H.: "Colorimetric detection of anthrax DNA with a peptide nucleic acid sandwich-hybridization assay", JOURNAL OF THE AMERICAN CHEMICAL SOCIETY, vol. 129, no. 27, July 2007 (2007-07), pages 8424-8425, XP002662762, ISSN: 0002-7863 -& Zhang N. and Appella D.H.: "Supporting Info", , 2007, XP002662763, Retrieved from the Internet: URL:http://pubs.acs.org/doi/suppl/10.1021/ ja072744j [retrieved on 2011-11-03]
- POKORSKI J.K ET AL.: "(S,S)-trans-cyclopentane-constrained peptide nucleic acids. a general backbone modification that improves binding affinity and sequence specificity.", JOURNAL OF THE AMERICAN CHEMICAL SOCIETY 24 NOV 2004 LNKD- PUBMED:15548003, vol. 126, no. 46, 24 November 2004 (2004-11-24), pages 15067-15073, XP002662764, ISSN: 0002-7863 -& Pokorski J.K. et al.: "Supporting Info", , 2004, XP002662765, Retrieved from the Internet: URL:http://pubs.acs.org/doi/suppl/10.1021/ ja046280q [retrieved on 2011-11-03]
- ENGLUND E.A. ET AL.: "PNA-DNA duplexes, triplexes, and quadruplexes are stabilized with trans-cyclopentane units", JOURNAL OF THE AMERICAN CHEMICAL SOCIETY, vol. 128, no. 51, 1 December 2006 (2006-12-01), pages 16456-16457, XP55010854, ISSN: 0002-7863, DOI: 10.1021/ja064317w -& Englund E.A. et al.: "Supporting Info", , 2006, XP002662766, Retrieved from the Internet: URL:http://pubs.acs.org/doi/suppl/10.1021/ ja064317w [retrieved on 2011-11-04]
- POKORSKI J.K. ET AL.: "Cyclopentane-modified PNA improves the sensitivity of nanoparticle-based scanometric DNA detection", CHEMICAL COMMUNICATIONS - CHEMCOM; [6015D], ROYAL SOCIETY OF CHEMISTRY, GB, no. 16, 28 April 2005 (2005-04-28), pages 2101-2103, XP002466654, ISSN: 1359-7345, DOI: 10.1039/B418383E -& Pokorski J.K. et al.: Supplementary Information , 2005, XP002662767, Retrieved from the Internet: URL:http://www.rsc.org/suppdata/cc/b4/b418 383e/ [retrieved on 2011-11-04]
- MYERS M.C. ET AL.: "A Cyclopentane Conformational Restraint for a Peptide Nucleic Acid: Design, Asymmetric Synthesis, and Improved Binding Affinity to DNA and RNA", ORGANIC LETTERS, AMERICAN CHEMICAL SOCIETY, US, vol. 5, no. 15, 1 January 2003 (2003-01-01), pages 2695-2698, XP002466653, ISSN: 1523-7060, DOI: 10.1021/OL0348811 -& Myers M.C. et al.: "Supporting Info", , 2003, XP002662768, Retrieved from the Internet: URL:http://pubs.acs.org/doi/suppl/10.1021/ ol0348811 [retrieved on 2011-11-04]
- ANONYMOUS: 'Supporting Information', [Online] 13 October 2008, XP055179909 Retrieved from the Internet: <URL:http://www.wiley-vch.de/contents/jc_22 68/2008/z800441_s.pdf> [retrieved on 2015-03-27]
- Venugopal Chenna ET AL: "A Simple Cytosine to G-Clamp Nucleobase Substitution Enables Chiral [gamma]-PNAs to Invade Mixed-Sequence Double-Helical B-form DNA", ChemBioChem, vol. 9, no. 15, 13 October 2008 (2008-10-13), pages 2388-2391, XP055179908, ISSN: 1439-4227, DOI: 10.1002/cbic.200800441
- E.A. ENGLUND AND D.H. APPELLA: "Gamma-substituted PNA oligomers in nonnucleic acid based molecular recognition", ABSTRACTS OF PAPERS AMERICAN CHEMICAL SOCIETY, vol. 238, August 2009 (2009-08), pages 24-ORGN, 238TH AMERICAN-CHEMICAL-SOCIETY NATIONAL MEETING; WASHINGTON, DC, USA; AUGUST 16 -20, 2009 ISSN: 0065-7727

## Description

### TECHNICAL FIELD

The instant invention concerns compositions comprising polynucleotide and polynucleobase strands which serve as multivalent display ligands.

### BACKGROUND

Multivalent (or polyvalent) interactions refer to the simultaneous binding of multiple ligands on the surface of one molecular entity to multiple receptors on another. The strength and specificity of multivalent interactions depends on the cumulative effect of all the ligands and all the receptors involved in the process. Within a multivalent array, a single, isolated ligand-receptor interaction may actually be weak; however, the combined effect of multiple ligand-receptor interactions can be very strong. Such multivalent interactions occur throughout biology, and are important in numerous processes, such as those involving receptors at the surfaces of cells. For example, cell attachment, wound healing, and the immune response are basic examples where multivalent interactions are important. Therefore, multivalent interactions can be directly linked to cancer metastasis, blood clotting, and the generation of antibodies from a vaccination (*see generally,* Mammen et al., Angew. Chem. Int. Ed., 37:2754-94 (1998)).

Mimicking multivalent interactions on a synthetic scaffold is challenging, especially when large numbers of ligands (such as 5 or more) need to be displayed. There are numerous synthetic scaffolds that have been developed, but there are significant limitations that remain. Ideally, a scaffold for the multivalent display of ligands should be easily manipulated to display anywhere from 1 up to about 200 ligands in a controlled manner. Well-defined synthetic scaffolds have been developed for the display of small numbers of ligands. Such systems are good because a single synthetic entity can be made and isolated, but it is rare that such systems display more than 5 ligands. Beyond this, well-defined synthetic scaffolds become very challenging to make. To study the multivalent effects of larger numbers of ligands, scientists rely on synthetic systems that are less well-defined and consist of mixtures. In this area, it is common to use polymers, dendrimers, proteins, and synthetic nanostructures (such as gold nanoparticles) as the synthetic scaffold to support larger numbers of ligands. Unfortunately, these larger systems are heterogeneous mixtures where the number of ligands per scaffold cannot be rigorously defined. In these cases, scientists determine an average number of ligands per scaffold or report the range of ligands per scaffold. Heterogeneous mixtures are often not acceptable by FDA standards for application as a therapeutic. As such, improved scaffolds are needed.

Englund et al. (Angew. Chern. Int. Ed. Engl., 2007, 46(9), 1414-1418) discloses peptide nucleic acid (PNA) comprising side chains extending from the gamma position of the aminoethylglycine backbone of the PNA. Chenna et al. (ChernBioChem, 2008, 9(15): 2388-2391) teaches that randomly folded, single-stranded PNAs can be preorganized into a right-handed helix by installation of an L-alanine derive, S chiral center at the gamma position of the aminoethylglycine backbone unit.

APPELLA D.H.: "Molecular designs for peptide nucleic acids in detection and multivalent platforms", ABSTRACTS OF PAPERS AMERICAN CHEMICAL SOCIETY, vol. 238, August 2009, page 62-ORGN, 238th MEETING OF THE DIVISION OF CHEMICAL TOXICOLOGY OF THE AMERICAN-CHEMICAL-SOCIETY, WASHINGTON, DC, USA; AUGUST 16-20, 2009

E.A. ENGLUND and D.H. APPELLA: "Gamma-substituted PNA oligomers in nonnucleic acid based molecular recognition", ABSTRACTS OF PAPERS AMERICAN CHEMICAL SOCIETY, vol. 238, August 2009 (2009-08), page 24-ORGN, 238th AMERICAN-CHEMICAL-SOCIETY NATIONAL MEETING; WASHINGTON, DC, USA; AUGUST 16 -20, 2009

ENGLUND E.A.: "Development of gamma-substituted PNA as versatile scaffolds for multivalent display", ABSTRACTS OF PAPERS AMERICAN CHEMICAL SOCIETY, vol. 237, March 2009, page 13-ORGN, 237th NATIONAL MEETING OF THE AMERICAN-CHEMICAL-SOCIETY; SALT LAKE CITY, UT, USA; MARCH 22-26, 2009

### SUMMARY

In some aspects, the invention concerns composition comprising: strands of polynucleotide and strands of PNA, each PNA strand comprising: (i) from 7 to 50 nucleobase subunits; and (ii) one or more gamma substituents; the PNA strands being complementary to at least a portion of at least some of the polynucleotide strands, and the molar ratio of PNA strands to polynucleotide strands being at least 2:1, wherein the gamma substituent is capable of binding to a protein. In some preferred embodiments, the polynucleotide strands are DNA. Some DNAs comprise at least 120 nucleotide subunits. In some embodiments the DNAs are single stranded. In other embodiments, RNA is the preferred polynucleotide. In some embodiments the RNAs are single stranded. Some RNAs comprise at least 120 nucleotide subunits. Certain embodiments contain PNA with a backbone having at least one cyclopentyl residue.

Some compositions of the invention have gamma substituents that are capable of binding to a receptor on the surface of a cell, binding to a cell surface molecule, or eliciting an immune response. In some embodiments, the molar ratio of PNA strands to polynucleotide strands is 2:1 to 10:1. In certain embodiments, the ratio is 3:1 to 7:1 or 4:1 to 6:1.

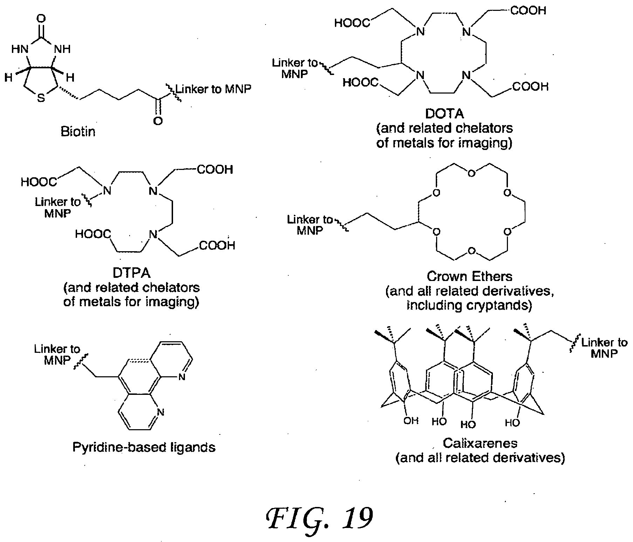

Preferred gamma substituents, independently, include -R-NX¹X², where: R is a C₁-C₁₂ alkyl; X¹ and X² are, independently, H, biomolecules, fluorescent groups, metal ligands, Michael acceptors, azides, alkynes, or thiols; where at least one of X¹ and X² are other than H. In certain embodiments, X¹ and X² are, independently, H, biotin, fluorescein, thiazole orange, acridine, pyrene, Alexafluor Dyes, polypeptide, sugars (such as mannose or lactose), nucleic acid derivatives, oligonucleotides, RGD (Arg-Gly-Asp) or cyclic RGD. Additional groups and ligands that may be attached to the multivalent nano peptide nucleic acid (MNP) for multivalent display include, but are not limited to, cyclodextrins, porphyrins, polyhedral cage compounds containing boron, and the compositions depicted in **Figure 19** (such as, biotin, 1,4,7,10-tetraazacyclododecane-N,N',N",N"-tetraacetic acid (DOTA), diethylene triamine pentaacetic acid (DTPA), a cryptand, a crown ether (12-crown-4, 15-crown-5, 18-crown-6, dibenzo-18-crown-6, and diaza-18-crown-6, or derivatives, for example), a pyridine-containing ligand, or calixarenes (such as calix[4]arenes, e.g., cone-4-tert-butylcalix[4]arenetetra(diethylamide), and calix[6]arenes). In some embodiments, derivatives of the aforementioned X¹ and X² groups and ligands may be utilized. In addition to gamma substituents, the N-terminal PNA residues of the compounds described herein can also include substituents (R2 and R3) on the nitrogens of the N-termini. Accordingly, a single PNA residue can have up to 3 substituents, one gamma substituent and two terminal substituents.

In some embodiments the individual PNA residues described herein can have one substituent. In some instances this substituent will be conjugated to the gamma carbon of the PNA residue. In some embodiments this substituent will be conjugated to the terminal nitrogen of the PNA residue. A PNA residue of this nature will be an individual residue in some embodiments. However, it may also be one of multiple PNAs in a larger strand. In some embodiments the PNAs described herein can be complexed with DNA to form an MNP.

In some embodiments the individual PNA residues described herein can have two substituents. In some instances at least one of the two substituents will be conjugated to the gamma carbon of the PNA residue. In some embodiments, at least one of the two substituents will be conjugated to a terminal nitrogen residue. In some instances at least one of the two substituents will be conjugated to the gamma carbon of the PNA residue and the other will be conjugated to a terminal nitrogen residue. A PNA residue of this nature will be an individual residue in some embodiments. However, it may also be one of multiple PNAs in a larger strand. In some embodiments the PNAs described herein can be complexed with DNA to form an MNP.

In some embodiments the individual PNA residues described herein can have three substituents. In some instances at least one of the substituents will be conjugated to the gamma carbon of the PNA residue. In some embodiments, two substituents will be conjugated to a terminal nitrogen residue. In some instances at least one of the substituents will be conjugated to the gamma carbon of the PNA residue and the other two will be conjugated to a terminal nitrogen residue. A PNA residue of this nature will be an individual residue in some embodiments. However, it may also be one of multiple PNAs in a larger strand. In some embodiments the PNAs described herein can be complexed with DNA to form an MNP.

The invention also concerns methods of treating or inhibiting a disease state in a mammal comprising administering to said mammal an effective amount of a compound described herein wherein at least some of the gamma substituents are selected to bind to a receptor on the surface of a cell associated with said disease state, to hinder the ability of a cell surface molecule to interact with a ligand that may trigger or prolong a disease state, or elicit an immune response.. In some embodiments, the disease state is related to, independently, cancer; infectious diseases caused by HIV, influenza, rhinovirus, rotavirus, *E*. coli, anthrax or cholera; diabetes (type 2), Chagas disease, chronic inflammatory diseases, and autoimmune diseases (see *generally,* Hecht et al., Curr. Opin. in Chem. Biol., 13:354-59 (2009) and Mammen *et al*.)..

In yet another aspect, the invention concerns methods of forming nanostructure platforms comprising contacting polynucleotide with PNA strands, wherein said PNA strands comprise:
(i) from 7 to 50 nucleobase subunits, and
(ii) one or more gamma substituents;
wherein the molar ratio of said PNA strands to said one or more polynucleotide strands is greater than 1:1 and said PNA strands are complementary to a portion of said polynucleotide strands. The PNA and DNA strands are as described herein.

### BRIEF DESCRIPTION OF THE DRAWINGS

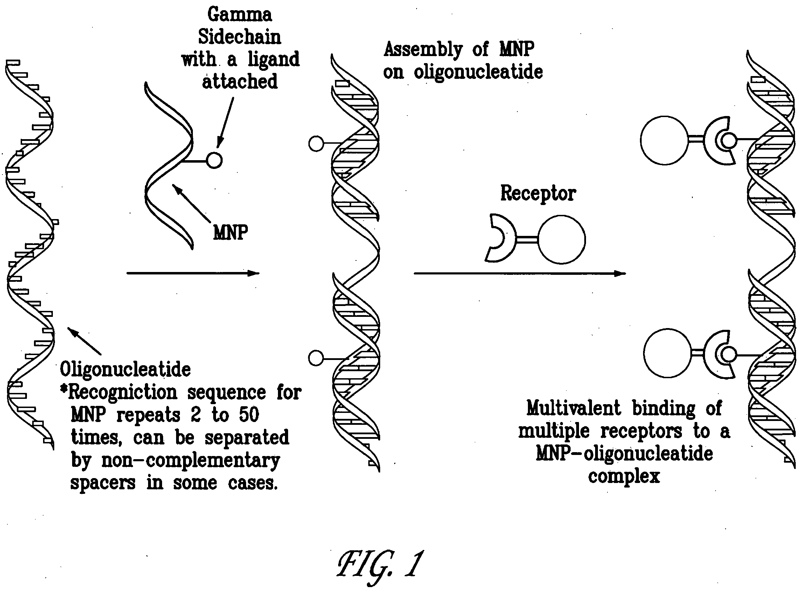

**Figure 1** presents a general scheme where 2 MNPs (each with one gamma sidechain) bind to a complementary oligonucleotide. More than one gamma sidechain, however, may be attached to the MNP (in other examples 2, 3, and 5 sidechains are attached, and more are possible). By using a longer oligonucleotide, more than just 2 MNPs may be assembled for multivalent display. Variation in the number of gamma sidechains and the sequence of the oligonucleotide can be used to create well-defined multivalent arrays of ligands attached to the MNP so that anywhere from 1 to 200 copies of the ligand are displayed. The distance between adjacent MNPs on the oligonucleotide can also be varied by altering the oligonucleotide's sequence to include segments that are non-complementary to the MNP.
**Figure 2** presents a general scheme where 2 different MNPs (each with a different nucleobase base sequence and each one with a gamma sidechain that has its own ligand) bind to a complementary oligonucleotide that has a sequence to recognize both MNP sequences. Again, more than one gamma sidechain may be attached to either MNP.
**Figure 3** shows constructs one side chain (no peg)PNA-(no peg)RGD and one side chain (peg)PNA-(no peg)RGD.
**Figure 4** shows constructs one side chain (peg)PNA-(peg)RGD and one side chain (long peg)PNA-(peg)RGD.
**Figure 5** shows a N-terminal side chain PNA-RGD construct.
**Figure 6** shows a two-side chain PNA-RGD construct.
**Figure 7** shows a three-side chain PNA-RGD construct.
**Figure 8** shows a three-side-chain PNA-poly mannose derivative.
**Figure 9** shows a five-side-chain PNA-Lactose construct.
**Figure 10** shows one side chain (no peg)PNA and one side chain (two peg linker)PNA constructs.
**Figure 11** shows N-Terminal Side Chain PNA construct.
**Figure 12** shows a two-side chain (two peg linkers) PNA construct.
**Figure 13** shows three-side chain (two peg linkers) PNA and five-side-chain PNA constructs.
**Figure 14** shows Tm data from PNAs with various gamma-sidechains.
**Figure 15** shows IC₅₀ data for various multivalent constructs.

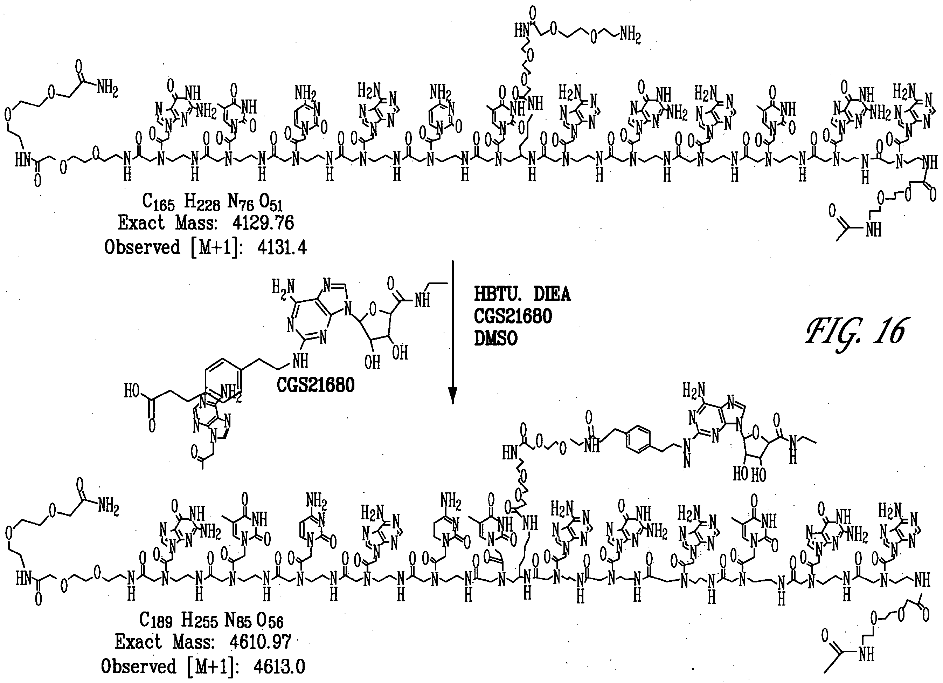

**Figure 16** presents a schematic for attaching an agonist for an adenosine receptor (A2A) to a PNA.

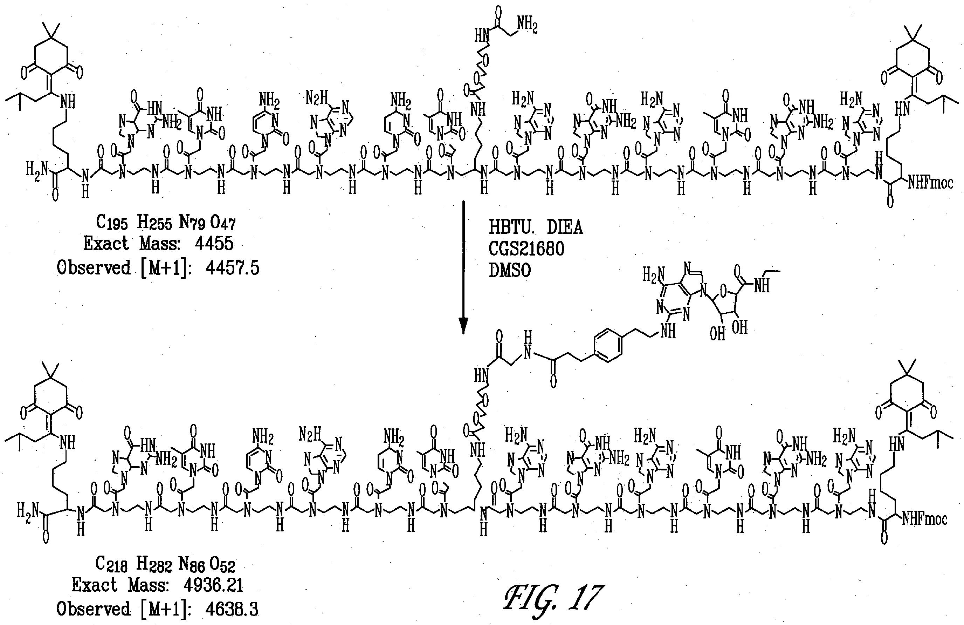

**Figure 17** presents a schematic for attaching an agonist for an adenosine receptor (A2A) to a PNA.

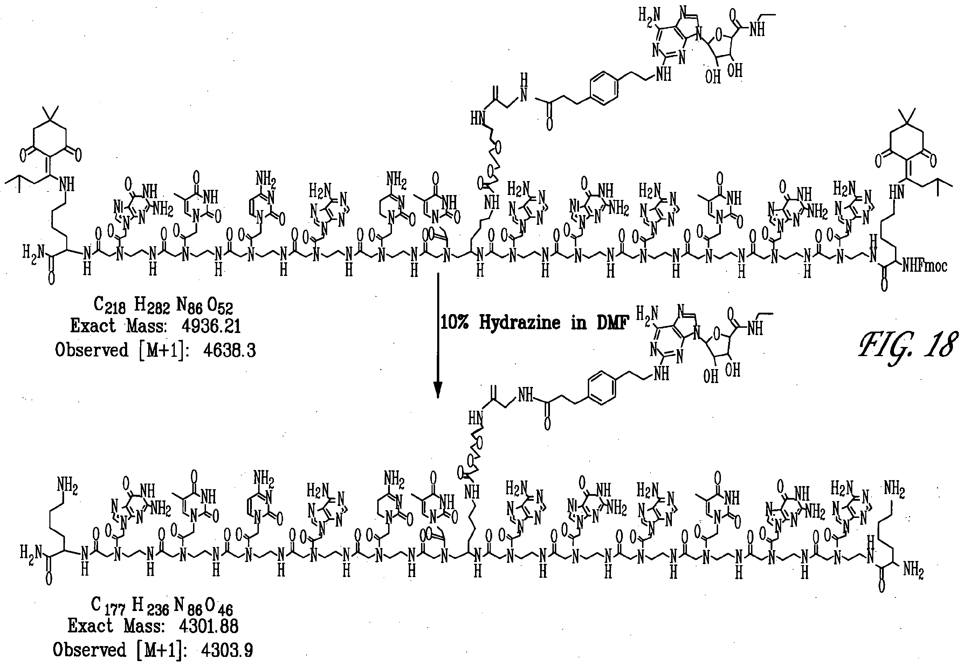

**Figure 18** presents a schematic for attaching an agonist for an adenosine receptor (A2A) to a PNA.
**Figure 19** presents structures of representative groups and ligands that can be attached to the MNP for multivalent display.
**Figure 20** shows metastatic tumor foci in mouse lungs for mice treated with either vehicle (negative control), monovalent cyclic RGD ligand, or an MNP conjugated to 15 cyclic RGD ligands.
**Figure 21** provides a graphical illustration of the tumor foci shown in figure 21.

### DETAILED DESCRIPTION OF ILLUSTRATIVE EMBODIMENTS

The technology of the present invention overcomes the barriers that persist for the current multivalent scaffolds. The technology uses peptide nucleic acids (PNAs) which are backbone substituted to provide a multivalent scaffold (MNP). Preferably in the MNP, one or more sidechains have been introduced at one or more gamma carbons of the PNA backbone.

PNAs are synthetic molecules that possess the bases derived from DNA. Similar to DNA, the sequence of bases on a PNA determines the complementary sequence of nucleic acids to which a PNA will bind. Preferred sidechains at the gamma carbon of PNA have a nitrogen atom to facilitate attachment of ligands to the sidechains that extend off of the backbone. The term "gamma-sidechain-modified PNAs as Multivalent Nano PNA" (MNP) is used to highlight that these molecules are being used for the multivalent display of ligands on a nanoscale synthetic scaffold.

The MNPs can interact with a polynucleotide to form a complex where the ratio of MNP to polynucleotide is greater than 1:1. Suitable polynucleotides include DNA, RNA, and even synthetic polynucleobases such as PNA. The DNA and RNA may be natural or synthetic constructs. While in some embodiments, the preferred polynucleotide is DNA, it is understood that when DNA is used in an example or to describe an embodiment of the invention, that other polynucleotides may be utilized instead of DNA. As used herein, the terms "polynucleotide" and "oligonucleotide" are used interchangeably. In some embodiments, oligonucletides have 200 or fewer bases.

In one application of the invention, MNP is used alone to target proteins. In this application, the arrangement of sidechains on the MNP can create a unique three-dimensional display that is guided in part by the intramolecular interactions of the nucleobases. Such a folded structure can be used as a binder to proteins, similar to aptamers. Aptamers are single-stranded oligonucleotides that are generated through an in vitro selection and evolution process called SELEX (which stands for Systematic Evolution of Ligands by Exponential enrichment). The evolution process for SELEX depends on employing several rounds of enzymatic amplification of oligonucleotide sequences that are selected to have good binding properties to a particular target. An example of a drug that has been developed from an aptamer is Macugen (Pegaptanib). Since aptamers composed of DNA and RNA are themselves susceptible to nuclease degradation, the field has made significant efforts to find synthetic derivatives that are sufficiently stable for therapeutic applications. While it is not possible to use the same evolution process for MNPs, library screening for protein binding may be used to identify MNPs that target proteins.

The MNPs described herein can be used to target protein. In some instances targeted proteins will be cell surface proteins. For example, cell surface proteins that are targets can be transmembrane proteins, lipid-anchored proteins, or peripheral proteins. Accordingly, the targeted proteins can be a cellular receptor or cellular adhesion molecule. In some instances the cell surface protein is an integrin. In some embodiments, the integrin may be α₁β₁, α₂β₁, α₄β₁, α₄β₇, α₅β₁, α₆β₁, α_{L}β₂, αMβ₂, α_{IIb}β₃, α_{V}β₃, α_{V}β₅, α_{V}β₆, or α₆β₄. In some embodiments described herein the disclosed MNPs may be used to bind to, or disrupt the activity of integrin α₆β₄. In some embodiments the targeted protein may be a G-coupled surface protein, such as a receptor. In other embodiments the cell surface proteins are ion channel linked receptors. In some embodiments the targeted protein may be an enzyme-linked receptor protein. In some aspects the targeted protein is a cadherin, such as E-cadherin, N-cadherin, cadherin 12, or P-cadherin. Selectins may also be targeted by the MNPs described herein. For example, E-selectin, P-selectin, or L-selectin may be targeted by the described MNPs. In some aspects, inter-cellular adhesion molecule 1 (ICAM-1) is targeted by the MNPs described herein. In other embodiments, sialic acid on the cell surface may be targeted. C-type lectins may also be targeted by the MNPs described herein. For example, some C-type lectins that may be targeted include: lecticans, asialoglycoprotein and DC receptors, collectins, NK cell receptors, multi-CTLD endocytic receptors, and thrombomodulin to name only a few. In addition, toll-like receptors may be targeted by the described MNPs. Some toll-like receptors that may be targeted include TLR 1, TLR 2, TLR 3, TLR 4, TLR 5, TLR 6, TLR 7, TLR 8, TLR 9, TLR 10, TLR 11, TLR 12, or TLR 13.

Another application of MNP is to assemble multiple copies on a single-stranded nucleic acid (such as DNA, RNA, or any other type of synthetic nucleic acid mimic, like PNA or locked nucleic acid or LNA). Assembly in this manner acts as the template to assemble multiple MNPs. For example, a MNP that consists of 12 bases and has one ligand attached to a sidechain, can display 10 of these ligands in a multivalent array by complexing this MNP to a 120-base DNA sequence that has the appropriate complementary sequence to the MNP. With this system, a library of different entities with different numbers of ligands can readily be produced. For instance, switching from displaying 10 ligands to 5 is easily accomplished by simply using a different (shorter) DNA and the same MNP. With this system, one is not restricted to just one ligand per MNP. The chemistry of the present invention allows attachment of multiple sidechains to the original PNA sequence so that, if desired, a sidechain can be attached at every position in the final MNP. In this case, a 12 base MNP with a ligand-bearing sidechain at every position in the backbone could be used to display 120 ligands if assembled onto a 120-base DNA sequence that has the complementary sequence repeated 10 times. The versatility and accuracy of the system of the present invention is unparalleled by current multivalent scaffolds because we can display anywhere from 1 up to about 200 ligands simply by proper selection of the number of sidechains on the MNP and the corresponding DNA (or other type of oligonucleotide) assembly sequence.

Another advantage of the system of the present invention is that one accurately knows the number of ligands displayed in each case because the interaction between MNP and DNA is well-defined. Furthermore, one can easily change the distance between adjacent ligands on the scaffold by inserting sections of non-complementary DNA sequences in between the MNP-binding portions of the DNA. An additional feature of this system is that it allows display of different types of ligands in a controlled, spatially-addressable manner. For example, if 2 different ligands (L1 and L2) each need to be displayed 3 times but in different specific orders (for example L1-L1-L1-L2-L2-L2 vs. L1-L2-L1-L2-L1-L2) this can be accomplished with the present system. To do this, one would make two different MNP nucleobase sequences (MNP1 and MNP2) and then attach L1 to the sidechain of MNP1 and L2 to the sidechain of MNP2. Examining the different order of ligands can be accomplished by making the appropriate DNA sequence to display the ligands in the desired order. This process can be extended to more ligands. For instance, to display 10 different substituents (L1, L2, L3, L4, L5, L6, L7, L8, L9, L10) one would design ten different MNPs, each with a unique polynucleobase sequence, and attach one ligand to each MNP. Each of the ten ligands could then be assembled onto a DNA sequence in a spatially-addressable manner. The technology uniquely allows small molecular rearrangements of multiple substituents to be explored. For the example of 10 substituents, the technology allows one to make ligand arrangements such as: L1-L2-L3-L4-L5-L6-L7-L8-L9-L10 or, reverse the position of L1 and L2, to give L2-L1-L3-L4-L5-L6-L7-L8-L9-L10. With the ability to make libraries of DNA, one can even explore all combinations of the 10 substituents. The exploration of substituent libraries in this manner could also be extended to DNA microarray technology as a way to assemble the substituent library and subsequently screen for activity and sequence information.

Those skilled in the art will understand that as many different substituents can be used as there are PNAs available for conjugation. Accordingly, the use of "10" substituents to illustrate the point of positional flexibility provided by the described system should not be seen as limiting. For example, MNPs described herein can be made of a PNA strand having 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 total substituents. In some embodiments all of the substituents on an MNP strand are the same. In other embodiments the substituents of the MNP strand differ. The degree to which substituents differ relative to one another can vary such that all of the substituents can be unique to the instance where about 5% are unique. Alternatively, about 10% of the substituents could be unique. In some embodiments about 15% of the substituents are unique. In some embodiments about 20% of the substituents are unique. In some embodiments about 25% of the substituents are unique. In some embodiments about 30% of the substituents are unique. In some embodiments about 35% of the substituents are unique. In some embodiments about 40% of the substituents are unique. In some embodiments about 45% of the substituents are unique. In some embodiments about 50% of the substituents are unique.

Additionally, with the multivalent assemblies of MNP-oligonucleotide complexes, crosslinking chemistry can be used to crosslink adjacent MNPs that are assembled onto the DNA. Covalent crosslinking of all (or some) the MNPs assembled on the DNA provides additional stability to the overall complex and likely provides stability for long-term storage.

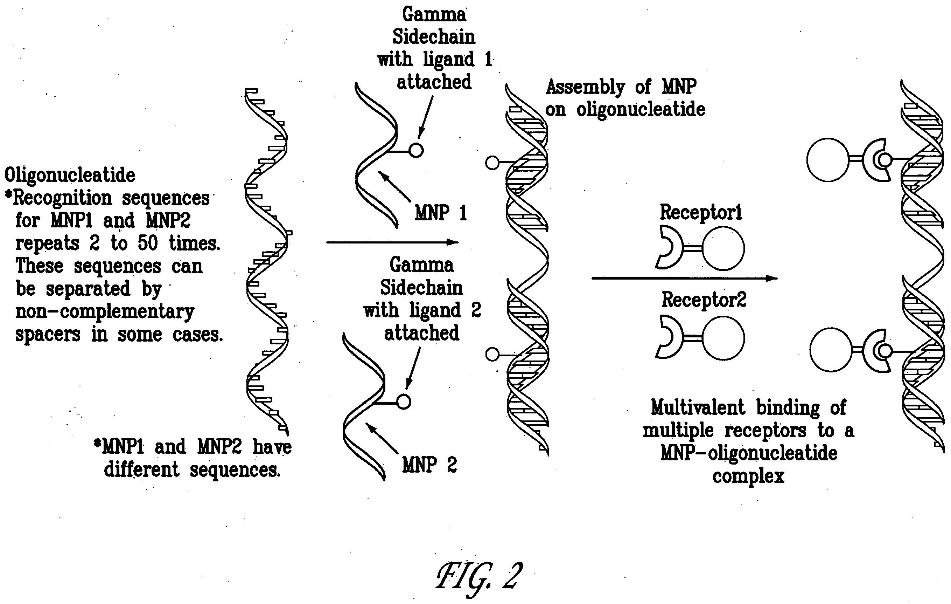

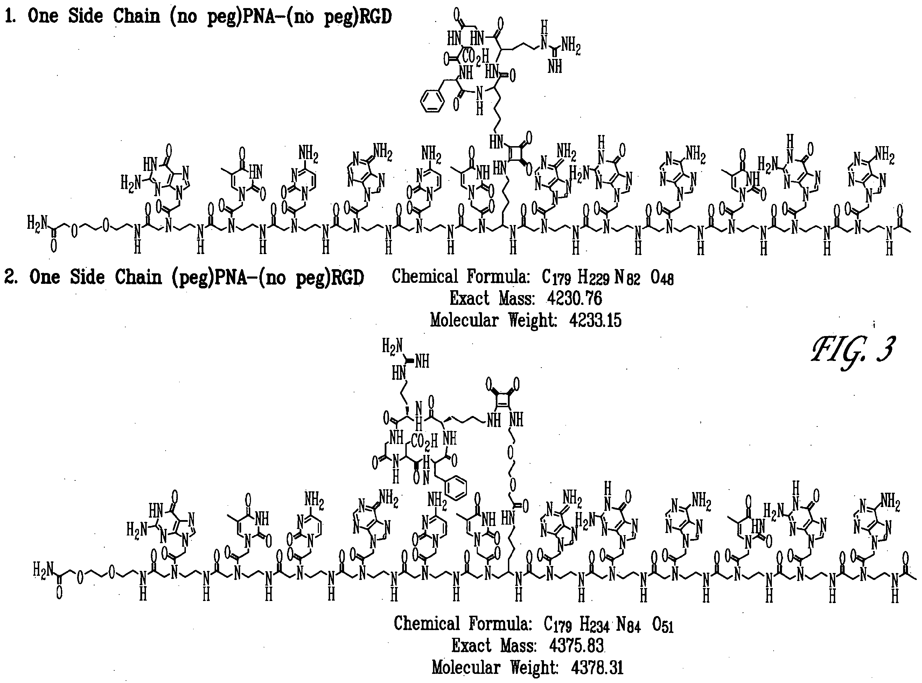

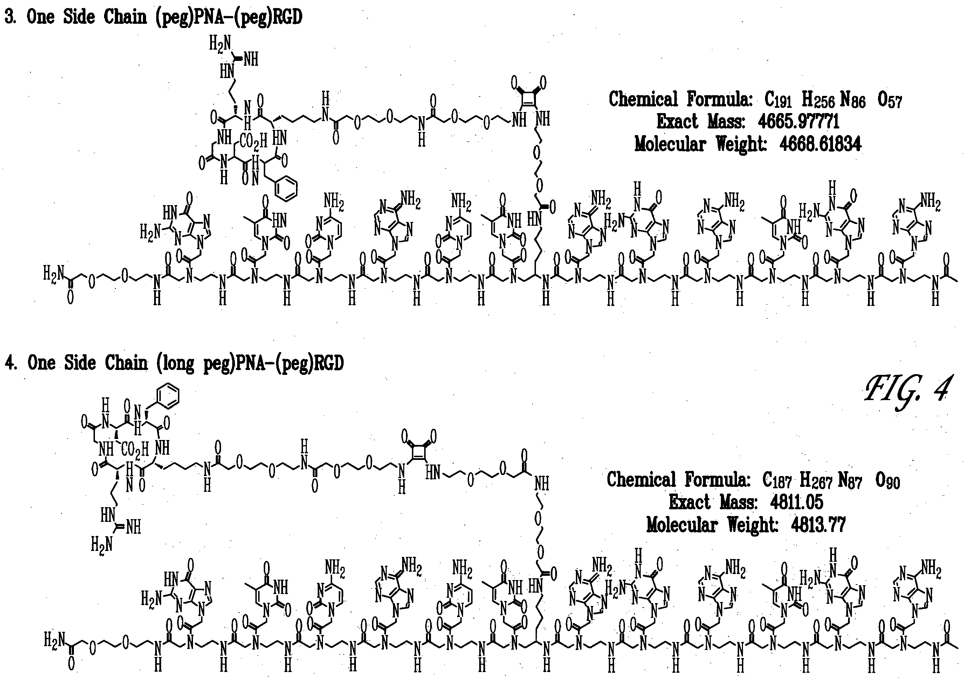

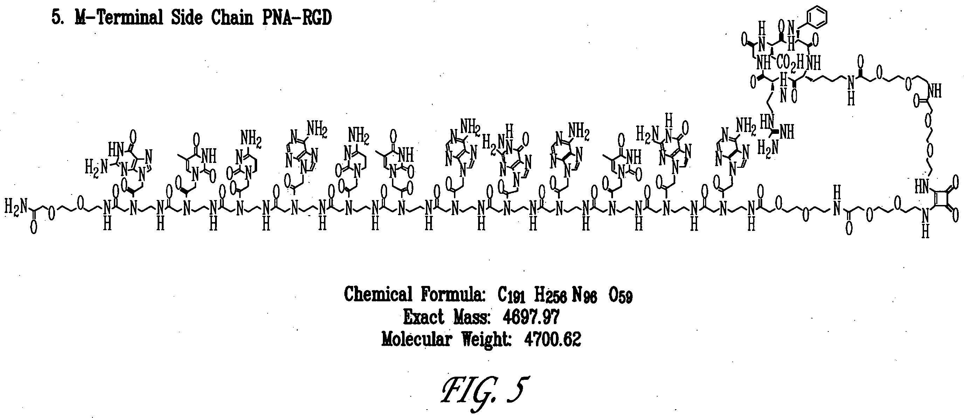

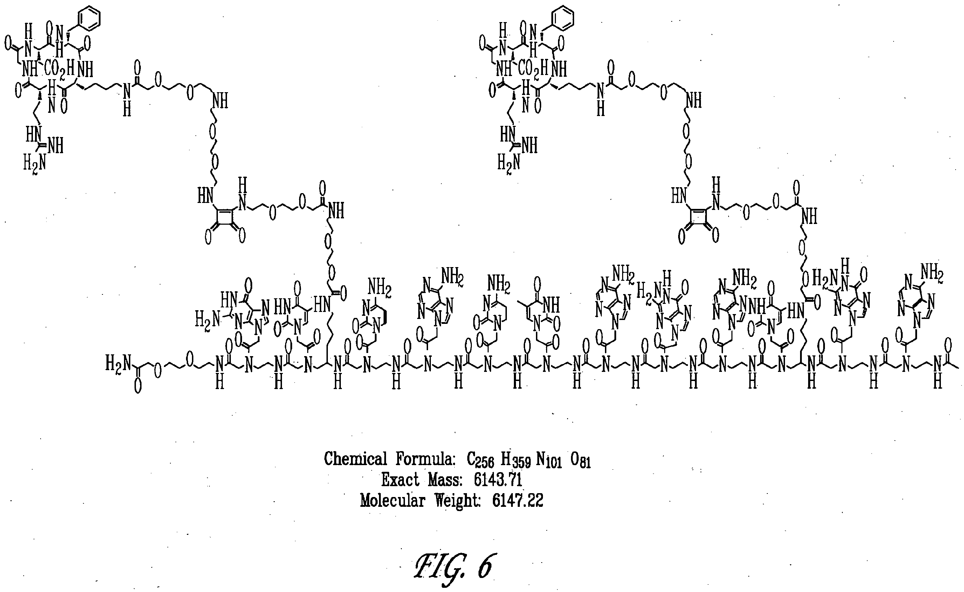

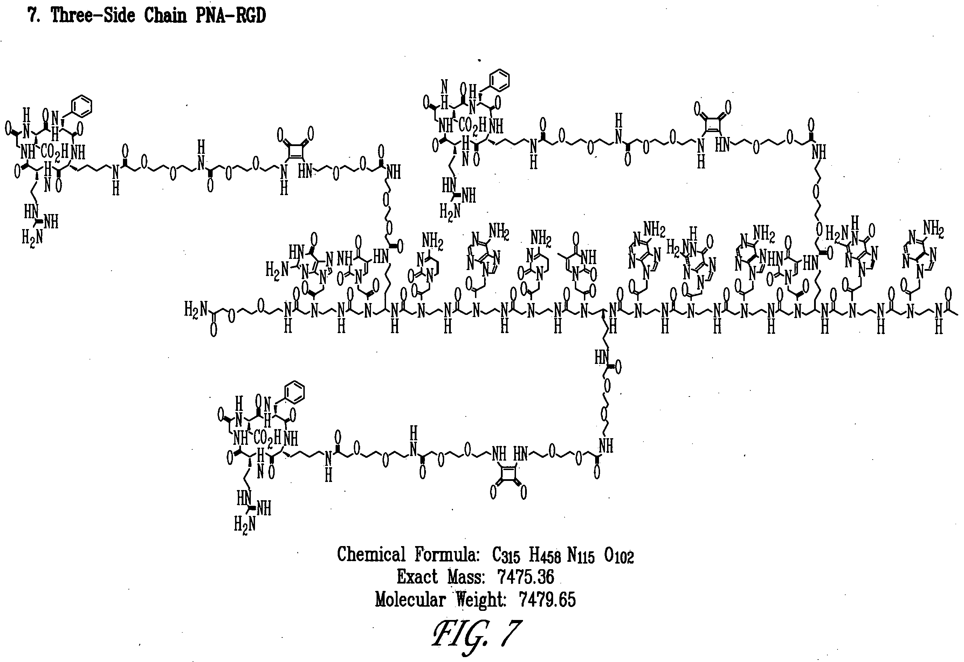

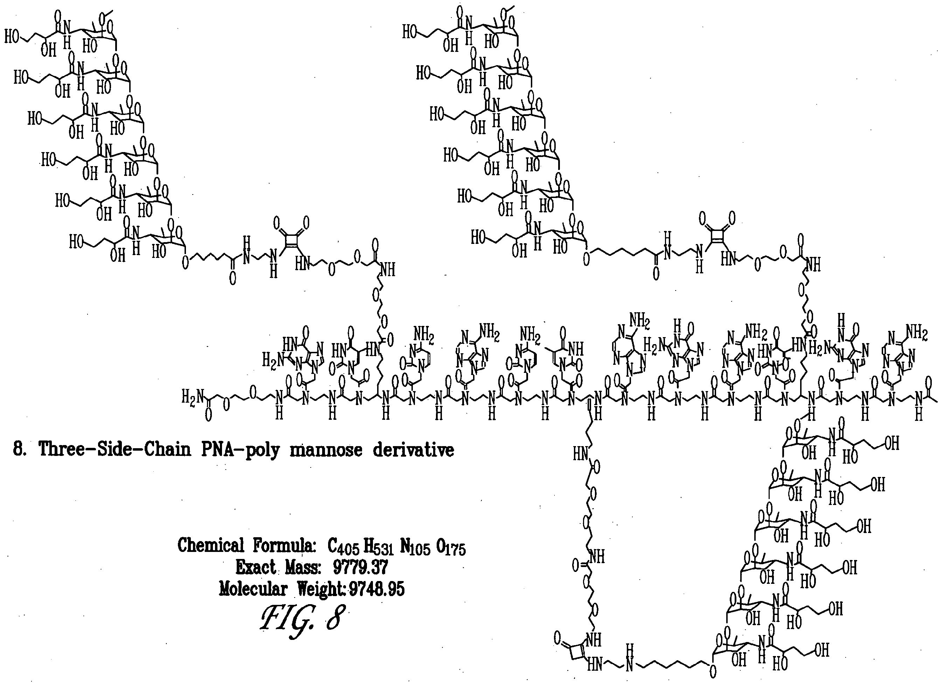

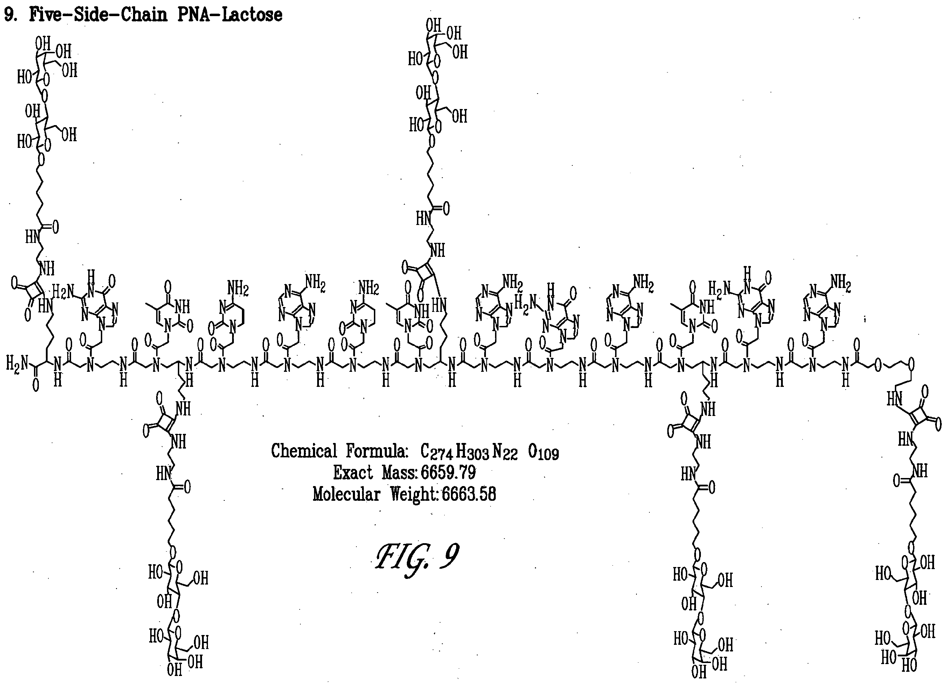

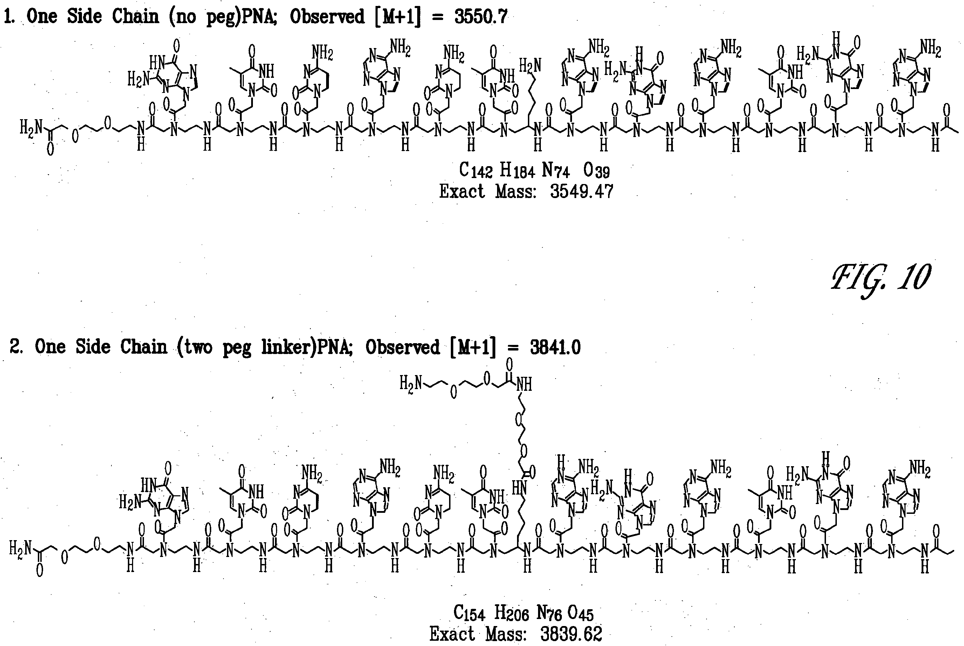

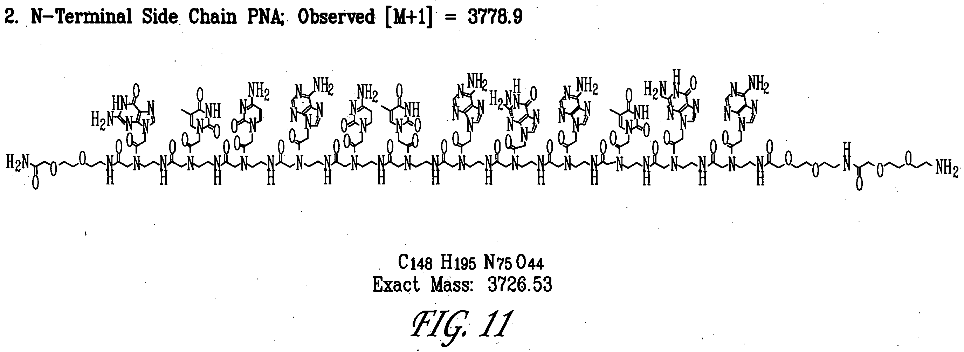

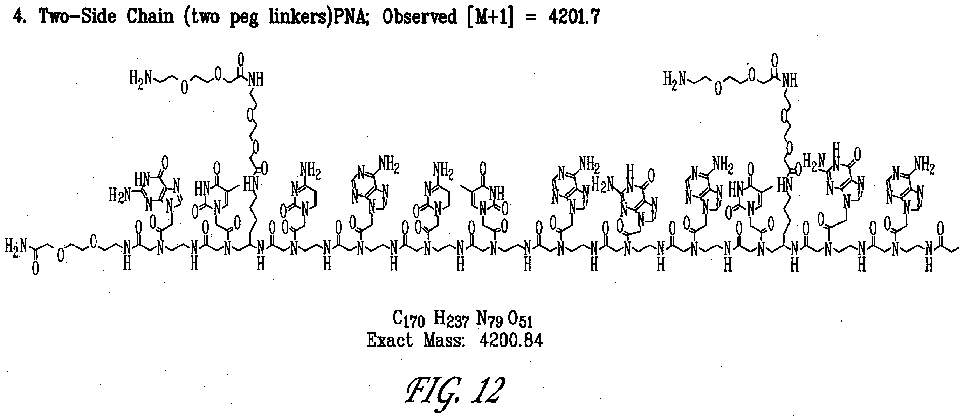

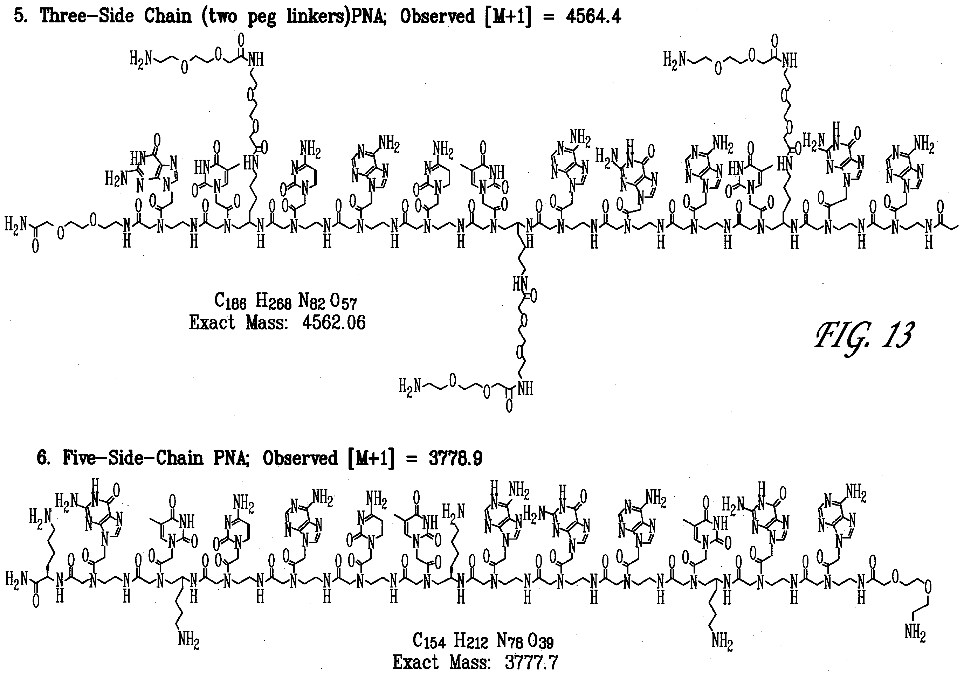

**Figures 2-13** show illustrative PNA constructs that have 1-5 side chains. Various side chains having moieties such as RGD and sugar derivatives (mannose and lactose, for example) are used in these examples. The structures in **Figures 10-13** can be used to attach a substituent via the terminal nitrogen-containing functional group on the side chain.

The present scaffolds can also feature multivalent assemblies of PNA-DNA complexes where adjacent PNAs that are assembled onto the DNA can be cross-linked. Covalent crosslinking of some or all of the PNAs assembled on the DNA would provide additional stability to the overall complex and likely provide stability for long-term storage.

As used herein, the term "MNP" refers to "multivalent nano PNA." MNP represents any PNA with a gamma sidechain in which there is a nitrogen atom somewhere in the sidechain. In some embodiments, MNP structure is represented by the following formula. 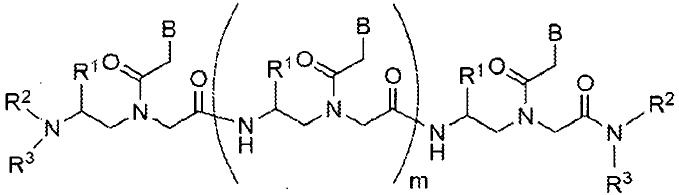 where R¹ is H or 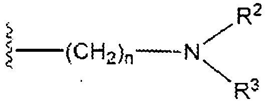 and m is 0 - 48, n is an integer from 1-5. B is a natural (A, T, G, C) or non-natural nucleobase (such as J, isoguanine, PPG).

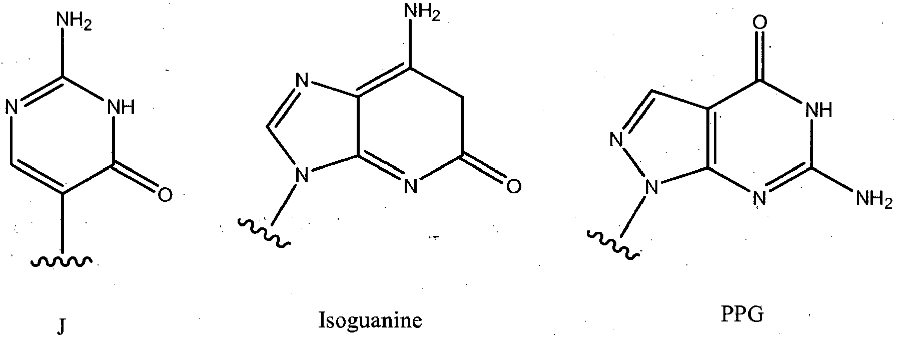

The range of groups for R² and R³ can vary from alkyl (such as CH₃ and derivatives such as substituted alkyls), to aryl (such as phenyl and obvious derivatives), to acyl (such as acetamido), to more complex linkers (such as PEG and associated variations) at the end of which are attached ligands for biomolecules, fluorescent groups, metal ligands, or other reactive groups (such as Michael acceptors, azides, alkynes, thiols) that may be used as a handle to attach other molecules. Some specific moieties that can be attached at the for R² and R³ positions include biotin, fluorescein, thiazole orange, acridine, pyrene, Alexafluor Dyes, polypeptides, sugars (such as lactose, mannose, or other oligosaccharides), nucleic acid derivatives (such as agonists or antagonists for adenosine receptors), oligonucleotides (such as G-quadruplexes). R² and R³ may also be peptide ligands such as RGD (Arg-Gly-Asp) and cyclic RGD.

In addition to the examples listed above, any substituent that is useful in the desired biological interaction may be utilized in the present invention. Such moieties can be added to the PNA by standard chemical reactions and methods discussed herein. Examples of substituents which can be placed in the gamma position include primary amines, hydrophobic groups, polar groups, hydrophilic groups, aromatic groups, peptide ligands, receptor agonists, sugars, imaging agents, or mixtures thereof.

In making the constructs of the present invention, gamma-substituents can be introduced based on their utility in interacting with specific receptors or other biological interaction sites. In a particular PNA, more than one substituent may be utilized. In a particular PNA/DNA scaffold, different PNAs may contain the same or different substituents depending on the target moiety.

In addition to the PNA depicted above, any of the numerous PNA variations that are known in the art can be utilized. Known PNA compositions include compositions represented by the following structures. 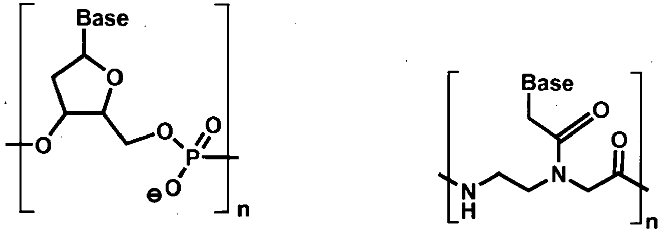 Natural and non-natural bases can be used in these structures and are well known by those skilled in the art.

Scheme 1 depicts a method of synthesis of a gamma-substituted monomer that can be used to make a PNA. The gamma substituent can serve as a point for further functionalization. These monomers can be converted to PNAs by methods known in the art. *See,* for example, Englund, E. A.; Appella, D. H. Angew. Chem. Int. Ed. Engl. 2007, 46, 1414.

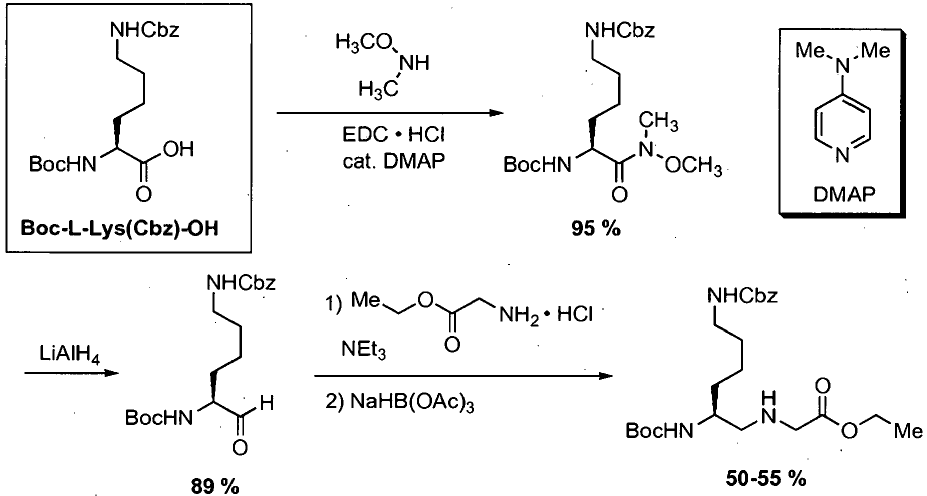

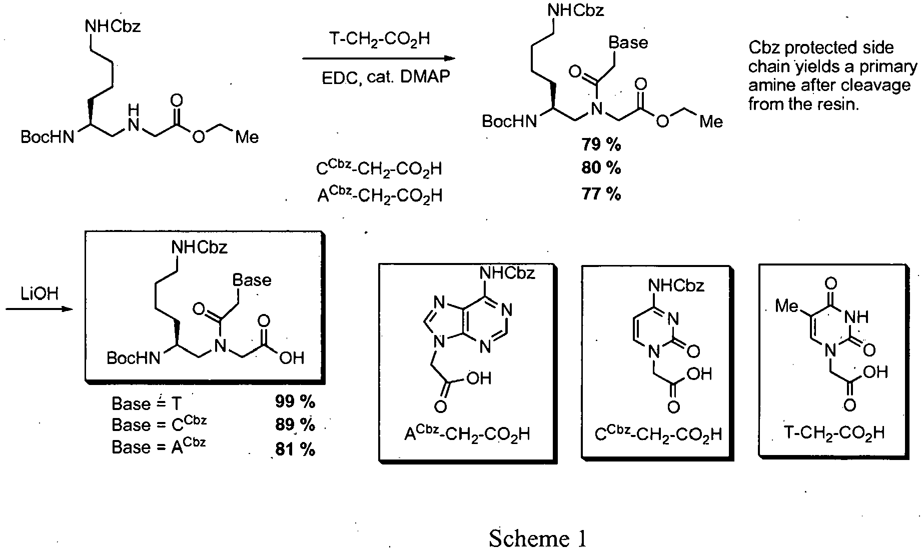

In scheme 2, use of FMoc allows for deprotection and coupling of amine while on resin. Reaction conditions adjusted to accommodate base labile Fmoc group. Typically, ester conversion to acid done via hydrogenolysis rather than hydrolysis. *See,* for example, Englund, E. A.; Appella, D. H. Org. Lett. 2005, 7, 3465.

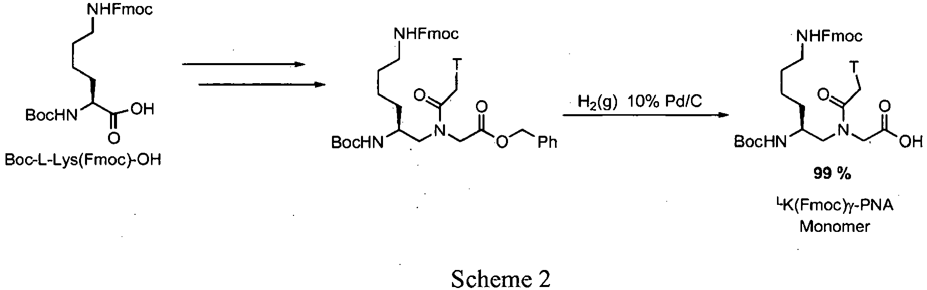

In Scheme 3, the oligomer is then cleaved from the resin (TfOH), purified via reverse phase HPLC, and characterized by mass spectrometry. *See,* for example, Koch, T.; et al. J. Peptide Res, 1997, 49, 80.

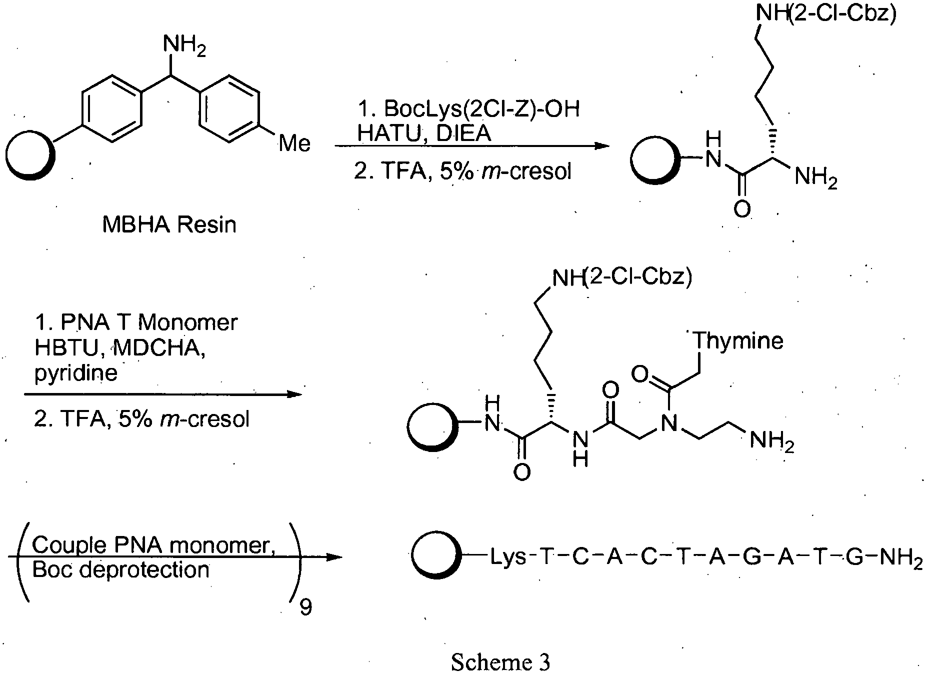

HBTU and HATU are defined by the structures below.

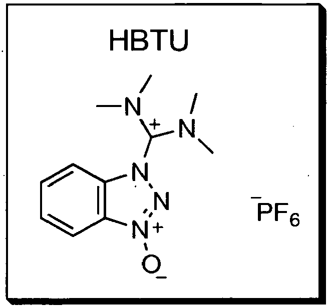

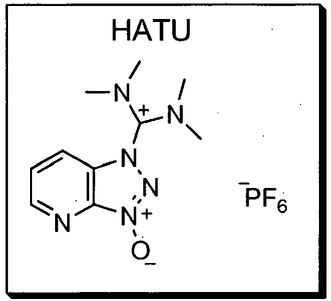

Sidechain modification of LK(Fmoc)γ-PNA is depicted in Scheme 4. This scheme provides access to wide variety of functionality. It is possible to conjugate some or all residues to different type of moiety via this process.

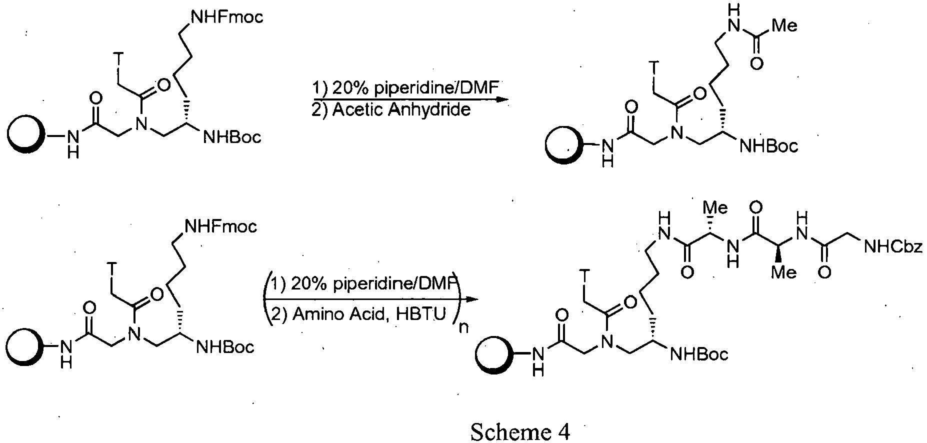

**Figure 2** depicts γ-substituted PNA oligomers containing multiple/various moieties and their use in forming a scaffold using multiple γ-substitutes PNAs on a larger DNA strand/construct. Using sequence specificity of PNA/DNA to control location and variety of conjugates allows for a specific order of substituents and/or the spatial position of such substituents. Any suitable PNA and DNA compositions may be utilized. Certain DNA sequences are commercially available. Other sequences can be synthesized by methods well known to those skilled in the art. PNAs can be synthesized by know methods, some of which are depicted herein.

As noted above, adjacent PNAs that are assembled onto the DNA can be cross-linked. Cross-linking reactive groups can be incorporated into PNAs by known techniques. In some embodiments, the cross-linking functional groups are attached in a terminal position in the PNA.

Cross-linking can be accomplished by use of cross-reactive functional groups. Many cross-reactive functional groups are known in the art and can be used with the present invention. In some embodiments, the cross-reactive functional groups can be of the formulas I and II. Reaction of a molecule of Group I with Group II produces a linkage shown by formula III.

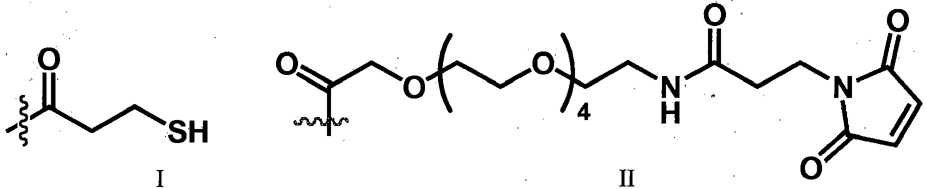

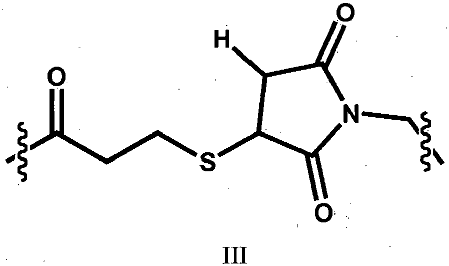

Some preferred PNAs contain *trans*-1,2-diaminocyclopentane which can potentially impact a broad range of scientific disciplines. Recent advances have improved the synthesis of *trans*-1,2-diaminocyclopentane. *See,* PCT Patent Application No. PCT/US2007/020466. These methods allow each nitrogen atom of cyclopentanediamine to be easily derivatized with identical or dissimilar groups. Incorporation of trans-1,2-diaminocyclopentane into Peptide Nucleic Acids (PNAs) has a beneficial effect on the recognition of DNA and RNA sequences.

Methods for PNA and cyclopentane-modified PNA synthesis and acquisition of melting temperature data can be found in Pokorski, et al., J. Am. Chem. Soc. 2004, 126, 15067. Addition of one or more cyclopentane groups into a PNA sequence improves the melting temperature to complementary DNA by ~5 °C per cyclopentane, regardless of which base is used.

The scaffolds can be made by contacting the PNAs with DNA (or other polynucleotide) under standard conditions known to those skilled in the art. *See,* for example, Englund, E. A.; Appella, D. H. Angew. Chem. Int. Ed. Engl. 2007, 46, 1414.

The scaffolds of the present invention can be utilized in pharmaceutical compositions. Such compositions can be produced by adding an effective amount of the scaffold composition to a suitable pharmaceutically acceptable diluent or carrier. Such carriers and diluents are will known to those skilled in the art.

The scaffolds and/or pharmaceutical compositions may be administered by methods well known to those skilled in the art. Such methods include local and systemic administration. In some embodiments, administration is topical. Such methods include ophthalmic administration and delivery to mucous membranes (including vaginal and rectal delivery), pulmonary (including inhalation of powders or aerosols; intratracheal, intranasal, epidermal and transdermal), oral or parenteral. Parenteral administration includes intravenous, intraarterial, subcutaneous, intraperitoneal or intramuscular injection or infusion; or intracranial (including intrathecal or intraventricular, administration).

Pharmaceutical compositions and formulations for topical administration include but are not limited to ointments, lotions, creams, transdermal patches, gels, drops, suppositories, sprays, liquids and powders. Utilization of conventional pharmaceutical carriers, oily bases, aqueous, powder, thickeners and the like may be used in the formulations.

The pharmaceutical compositions may also be administered in tablets, capsules, gel capsules, and the like.

Penetration enhancers may also be used in the instant pharmaceutical compositions. Such enhancers include surfactants, fatty acids, bile salts, chelating agents, and non-chelating non-surfactants. Such enhancers are generally described in U.S. Patent No. 6,287,860.

The scaffolds of the present invention find utility against diseases such as cancer (preventing metastasis, for example) and inhibition of HIV (by preventing attachment to the target cell's surface, for example). Other diseases include diabetes (Type 2), Chagas disease, chronic inflammatory diseases (such as celiac disease, vasculitis, lupus, chronic obstructive pulmonary disease, irritable bowel disease, atherosclerosis, arthritis, and psoriasis) and autoimmune diseases (such as diabetes mellitus type 1, Kawasaki disease, Graves' disease, Scleroderma).

In other applications, the scaffolds of the present invention can be utilized to generate vaccines. In one embodiment, a vaccine agent can be attached to a MNP of the instant invention. The vaccine agent can be a moiety that resembles a disease-causing microorganism such as a weakened or killed forms of the microbe or its toxins. Attachment to the MNP can be made using standard chemical techniques at positions of the MNP discussed herein. For example, antigens from anthrax and cholera can be attached to the scaffolds, which can be combined with an adjuvant to stimulate antibody production. The compositions of the instant invention can be used in treating a disease state or can be used prophylactically:

Another example of a vaccine that can be produced from the inventive compositions would be functionally equivalent or similar to Prevnar® (also known as Prevenar® in some countries). Prevnar® is a vaccine produced by Wyeth and marketed by Pfizer which protects humans (typically administered at 2, 4, 5, and 12-15 months of age) from certain pneumococcal bacteria that can cause serious diseases such as meningitis and bacteremia.

Prevnar® is a heptavalent vaccine, meaning it has seven different carbohydrates from seven different serotypes. The seven serotypes (strains) of S. pneumoniae included in the vaccine (4, 6B, 9V, 14, 18C, 19F, and 23F) were the strains that most commonly caused these serious diseases in children prior to the introduction of the vaccine. These carbohydrates, which can be derived from pneumococcus, are attached to a carrier protein to produce the vaccine. One application of the instant technology would be replace the carrier protein of the vaccine with a MNP of the present invention. In one embodiment, the same carbohydrates used in the commercial vaccine could be linked to the MNP. In practice, different carbohydrates could easily be attached to different MNP sequences. Using the instant MNPs, seven, or even more, different carbohydrates could be attached to the MNP if desired to increase the number of strains represented in the vaccine.

Other vaccines could similarly be made by appending agents that resemble a disease-causing microorganism such as a weakened or killed forms of the microbe or its toxins to the MNPs. Common vaccines include, but are not limited to, various strains of influenza vaccines, various strains of hepatitis vaccines, cholera vaccine, bubonic plague vaccine, polio vaccine, yellow fever vaccine, measles vaccine, rubella vaccine, tetanus vaccube, diphtheria, vaccine, mumps vaccine, typhoid vaccine, tuberculosis vaccine and rabies vaccine. Such vaccines could be produced by appending the appropriate agent to a MNP of the instant invention. Vaccines to other disease states can be produced by attaching the appropriate agent to a MNP. Additionally, vaccines to multiple conditions can be made by appending multiple agents to the MNP.

Provided herein are methods of treating or inhibiting a disease state in a mammal by administering to the mammal a therapeutically effective amount of one or more described MNPs having a substituent capable of binding a cell surface protein. In some embodiments the cell surface protein is a transmembrane protein, lipid-anchored protein, peripheral protein, a cellular receptor or an adhesion protein. In some aspects the mammal can be a rodent (mouse or rat), equine, feline, canine, or primate. In some embodiments, the described primate can be a human.

In some embodiments, the described methods therapeutic methods directed to reducing metastasis in a mammal. In some embodiments these methods are carried out by administering to the mammal a therapeutically effective amount of one or more described MNPs having a substituent capable of binding a cell surface protein. In particular embodiments the MNP administered to reduce metastasis is an MNP strand having 15 cyclo-RGD gamma substituents. In some aspects the mammal can be a rodent (mouse or rat), equine, feline, canine, or primate. In some embodiments, the described primate can be a mouse. In some embodiments, the described primate can be a human.

Also described are methods for detecting the presence of a cell surface protein using the described MNPs. In some embodiments the described method can be carried out by administering to a subject a described MNP capable of binding to the cellular protein target of interest and detecting the administered MNP. To facilitate detection, in some embodiments the MNP may be labeled with a reporter molecule. For example, the MNP may be radio labeled, conjugated to a fluorescent label, biotinylated, conjugated to DOTA, DTPA, or consist of a radionuclide. Other acceptable labels are widely known within the art. In some aspects the subject may be a rodent (mouse or rat), equine, feline, canine, or primate. In some embodiments, the described primate can be a human.

As used herein, the term "reporter molecule" is to be understood to mean any group which is detectable by analytical means *in vitro* and/*or in vivo* and which confers this property to his property to the conjugate. Some reporter molecules are a fluorescent molecule having fluorescence properties which are a function of the concentration of the molecule. Other reporter molecules have an absorbance spectra that can be monitored for detection. Numerous reporter molecules are known to those skilled in the art and are suitable for use with the present invention. One preferred reporter molecule is biotin.

The term "cross reactive groups" refers to at least two groups that are capable of reacting to form a covalent bond linking the first and second PNAs.

As used herein, the terms "a", "an", "the" and the like refer to both the singular and plural unless the context clearly indicates otherwise.

Also as used herein, the description of one or more method steps does not preclude the presence of additional method steps before or after the combined recited steps. Additional steps may also be intervening steps to those described. In addition, it is understood that the lettering or order of process steps or ingredients is a convenient means for identifying discrete activities or ingredients and the recited lettering can be arranged in any reasonable sequence.

Where a range of numbers is presented in the application, it is understood that the range includes all integers and fractions thereof between the stated range limits. A range of numbers expressly includes numbers less than the stated endpoints and those in-between the stated range. A range of from 1-3, for example, includes the integers one, two, and three as well as any fractions that reside between these integers.

### EXPERIMENTAL SECTION

The following examples are intended to be illustrative and not limiting in nature.

General: Proton nuclear magnetic resonances (¹H NMR) and ¹³C nuclear magnetic resonances (¹³C NMR) were recorded in deuterated solvents on an iNOVA (500 MHz) and a Bruker (300 MHz) spectrometer. Chemical shifts are reported in parts per million (ppm, δ) relative to tetramethylsilane (δ 0.00) or residual proto solvent DMSO (δ, 2.50). ¹H NMR splitting patterns are designated as singlet (s), doublet (d), triplet (t), or doublet of doublets (dd). Splitting patterns that could not be interpreted or easily visualized are designated as multiplet (m). Electrospray mass spectra (ESI-MS) were obtained using a Micromass Quattro II Triple Quadrupole HPLC/MS/MS Mass Spectrometer. Matrix assisted laser desorption/ionization mass spectra were obtained using a PerSeptive Biosystems Voyager DE MALDI-TOF system with 2',4',6'-trihydroxyacetophenone monohydrate matrix. Analytical thin-layer chromatography (TLC) was carried out on Sorbent Technologies TLC plates precoated with silica gel (250 mm layer thickness). Flash column chromatography was performed using a Biotage Flash12+ apparatus on Biotage Si 25+M columns. Solvent mixtures used for TLC and flash column chromatography are reported in v/v ratios. Unless otherwise noted, all commercially available reagents and solvents were purchased from Aldrich and used without further purification. BocLys(Cbz)-OH, and EDC were purchased from Advanced ChemTech. Thymine acetic acid (T-CH₂-CO₂H) was purchased from Aldrich. CCbz-CH₂-CO₂H and ACbz-CH₂-CO₂H were synthesized by PolyOrg Inc. DMF, dichloromethane, and THF were passed through activated alumina on a solvent purification system made by Glass Contour. Unless otherwise indicated, all reactions were performed under an inert atmosphere of N². Glassware was dried in an oven at 180 °C for at least 2 hours prior to use.

### Example 1: Synthesis and Characterization of PNA conjugates.

A solution of gamma-amino side chain peptide nucleic acid (γ-PNA, 1.5 µmol), 3,4-diethoxy-3-cyclobutene-1,2-dione (45 µmol, 30 equiv), triethylamine (90 µmol, 60 equiv), 500 µL of anhydrous dimethylsulfoxide (DMSO) and 250 µL of absolute ethanol was mixed for 3 hour. The solution was concentrated and the residue was washed with diethyl ether (3×1.0 ml), and dried under vacuum to get squaric acid-conjugated PNA intermediate as a white solid. The above PNA intermediate was added to a solution of amino group-bearing RGD or carbohydrate monomer (30 µmol, 20 equiv), triethylamine (45 µmol, 30 equiv) in 500 µL of anhydrous DMSO and 250 µL of absolute ethanol. After 18 hours of shaking, the solution was concentrated. The residue was purified by reversed-phase HPLC to obtain the final conjugate product (57%~90% yield over two steps) as a white solid.

Reverse-phase HPLC experiments were conducted with 19 x 150 mm Waters C8 reverse phase column using a Agilent HPLC. The typical flow rate was 12 mL/min. HPLC buffers consisted of 0.1% aqueous trifluoroacetic acid and acetonitrile.

**Table 1. Mass spectrometry results of some PNA conjugates. LCMS data was obtained on an Agilent 1200 series quadrupole LC/MSD electrospray trap.**

| PNA conjugate | Chemical Formula | Expected [M+H]⁺ | Found [M+H]⁺ |
|---|---|---|---|
| 1 | C₁₇₃H₂₂₃N₈₃O₄₈ | 4231.8 | 4229.3 |
| 2 | C₁₇₉H₂₃₄N₈₄O₅₁ | 4376.8 | 4372.7 |
| 3 | C₁₉₁H₂₅₆N₈₆O₅₇ | 4667.0 | 4669.0 |
| 4 | C₁₉₇H₂₆₇N₈₇O₆₀ | 4812.0 | 4811.7 |
| 5 | C₁₉₁H₂₅₆N₈₆O₅₉ | 4699.0 | 4698.8 |
| 6 | Q₅₆H₃₅₉N₁₀₁O₈₁ | 6144.7 | 6144.6 |
| 7 | C₃₁₅H₄₅₀N₁₁₅O₁₀₂ | 7476.4 | 7477.6 |
| 9 | C₂₇₄H₃₉₂N₈₈O₁₀₉ | 6659.8 | 6662.0 |

### Example 2: Assembling PNA-DNA complex.

To a PBS buffer solution (1×) of 20 µM DNA, appropriate concentration of PNA conjugate was added at 25 °C depending on the repeat unit number on the DNA. The solution was heated to 92 °C and hold for 5 min, and then slowly cooled down to 25 °C over a period of 2 h.

### Example 3: Variation of Tm with Addition of Gamma-substituents

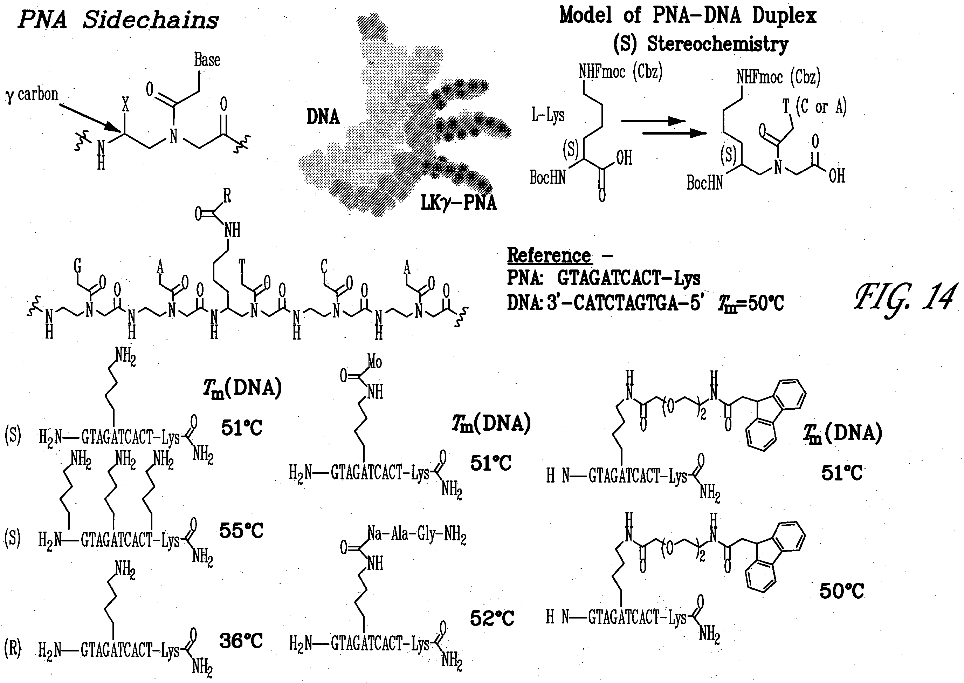

Various PNAs with gamma-substituents are depicted in **Figure 14**. The Tm values for these compositions were measured and addition of the sidechains caused Tm to increase. PNAs were made by standard methods. *See,* Englund, E. A.; Appella, D. H. Angew. Chem. Int. Ed. Engl. 2007, 46, 1414.

### Example 4: IC₅₀ Data-Cell-Adhesion Assay

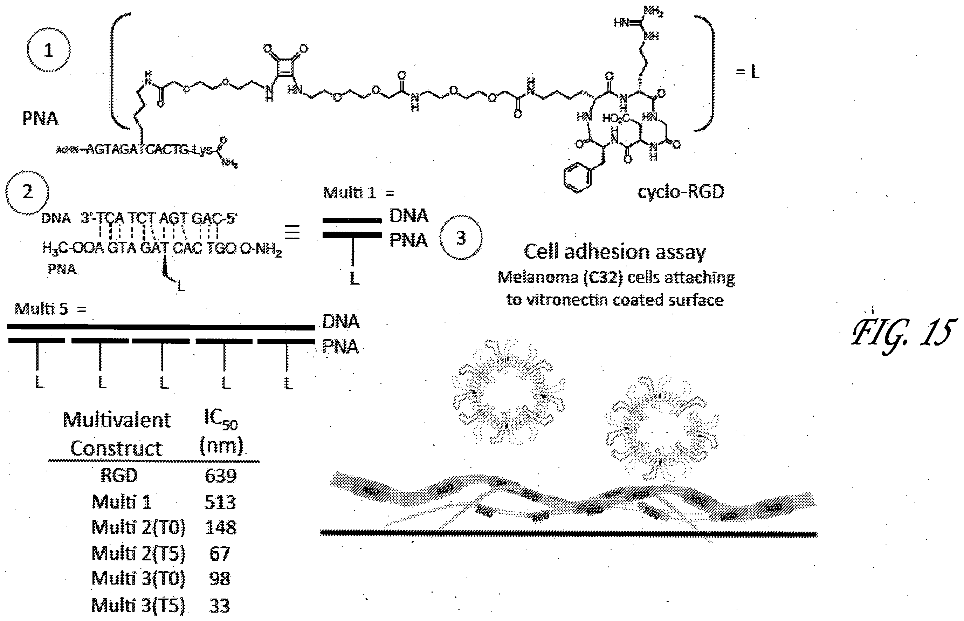

**Figure 15** and **Table 2** show IC₅₀ data for various multivalent constructs. Multi 1 indicates one PNA construct associated with the DNA. Multi 2 indicates two PNA construct associated with the DNA and so on. Constructs were synthesized by standard methods and are depicted in **Figure 15**. In the constructs depicted herein, the designator in parentheses (T5, for example) represents the number of thymine bases in the DNA sequence that is inserted to create a spacer between adjacent PNA binding portions of the DNA. T0 represents the absence of any spacer thymine bases. The number of thymine bases can vary widely (as illustrated in Table 3) and can be used to optimize the spacing between ligands displayed from an MNP.

**Table 2. IC₅₀ Data**

| Multivalent Construct | IC₅₀ (nM) |
|---|---|
| RGD | 639 |
| Multi 1 | 513 |
| Multi 2 (T0) | 148 |
| Multi 2 (T5) | 67 |
| Multi 3 (T0) | 98 |
| Multi 3 (T5) | 33 |

### Example 5: IC₅₀ Data for Various Constructs

IC₅₀ (nM) is reported in **Table 3** for various constructs. Each data point is the result of the assay performed in triplicate by the method described in Sipes, *et al,* J.

Biological Chem 1999, 274, 22755-22762. 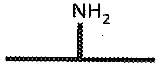 was used as a control. Control results are listed in parenthesis. "No ref" indicates that a control was not run for that particular experiments. "x" indicates no data is available for that experiment. RGD control for each data point (unnormalized)- C32 cells.

**Table 3. IC₅₀ Data (nM) for Various Constructs**

| | | | | |
|---|---|---|---|---|
| | 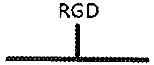 | 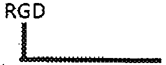 | 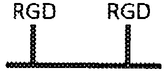 | 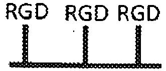 |
| Multi 1 | 513 (638), 232 (370), 284 (329), 457 (690) | 238 (370) | 54 (370), 111 (324) | 36 (370) |
| Multi 2-T0 | 147 (638), 130 (690) | 59.1 (561) | 23(324) | 22 (528) |
| Multi 2-T5 | 67 (638) | x | x | x |
| Multi 2-T10 | 186 (no ref) | x | x | x |
| Multi 2-T20 | 289 (no ref) | x | x | x |
| Multi 2-T40 | 160, 177 (no refs) | x | x | x |
| Multi 2-T60 | 158, 289 (no refs) | x | x | x |
| Multi 2-T80 | 128, 269 (no refs) | x | x | x |
| Multi 3 | 98 (638), 115 (690) | 44.8 (561) | 18 (324) | 15 (528) |
| Multi 3-T5 | 33 (638) | x | x | x |
| Multi 4-T10 | 23 (638), 42 (690) | 40.3 (561) | 19(324) | 14 (528) |
| Multi 4-T5 | 25 (638) | x | x | x |
| Multi 5 | 20 (370), 25 (329), 35 (690, 39 (no ref) | 16 (370) | 5 (370), 18 (324) | 3 (370) |
| Multi 6 | 30 (690), 17 (no ref) | 21.3 (561) | 9(324) | 8 (528) |
| Multi 7 | 15 (329), 25 (690), 14 (no ref) | 29.1 (561) | 7 (324) | 4 (528) |
| Multi 8 | 25 (690) | 16.9(561) | 6 (324) | 4 (528) |
| Multi 9 | 5 (329), 9 (no ref) | x | 3 (475) | 3 (528) |
| Multi 10 | 7 (no ref) | x | x | x |
| Multi 11 | 4 (329) | x | 3 (475) | 2 (528) |
| Multi 12 | 4(no rev) | x | x | x |
| Multi 13 | 3 (329) | x | x | x |
| Multi 14 | 3 (329) | x | x | x |
| Multi 15 | 2 (329) | x | 2 (475) | x |

### Example 6. Normalized IC₅₀ Data (C32 Cells) for Various Constructs

**Table 4** presents normalized IC50 (nM) data (C32 cells) IC50 data (nM) for various constructs. Constructs from Example 5 were utilized herein. The data presented in Table 4 are the information from Example 5 which have been normalized to the RGD control where the IC50 is 690 nM. This normalization allows for all the experiments in Example 5 to be directly compared to one another even though the experiments were run on different days and the values of the RGD control change.

**Table 4. Normalized IC₅₀ Data (C32 Cells) for Various Constructs**

| | | | | | | | | | | | | | | |
|---|---|---|---|---|---|---|---|---|---|---|---|---|---|---|
| | -------Central RGD--------- | | | | | N-term RGD | | | ------Two RGDs--- | | | | Three RGDs | |
| Norm Factor | 1 | 1.082 | 1.865 | 2.097 | | 1.23 | 1.865 | | 1.865 | 2.13 | 1.453 | | 1.307 | 1.865 |
| RGD only | 690 | 690 | 690 | 690 | | 690 | 690 | | 690 | 690 | 690 | | 690 | 690 |
| Multi 1 | 457 | 555 | 433 | 596 | | x | 444 | | 101 | 236 | x | | x | 67 |
| Multi 2-TO | 130 | 159 | x | x | | 73 | x | | x | 49 | x | | 29 | x |
| Multi 2-T5 | x | 72 | x | x | | x | x | | x | x | x | | x | x |
| Multi 3-T0 | 115 | 106 | x | x | | 55 | x | | x | 38 | x | | 20 | x |
| Multi 3-T5 | x | 36 | x | x | | x | x | | x | x | x | | x | x |
| Multi 4-T0 | 42 | 25 | x | x | | 49 | x | | x | 40 | x | | 18 | x |
| Multi 4-T5 | x | 27 | x | x | | x | x | | x | x | x | | x | x |
| Multi 5-T0 | 35 | x | 37 | 52 | | x | 30 | | 9 | 38 | x | | x | 6 |
| Multi 6-T0 | 30 | x | x | x | | 26 | x | | x | 19 | x | | 10 | x |
| Multi 7-T0 | 25 | x | x | 31 | | 36 | x | | x | 15 | x | | 5 | x |
| Multi 8-T0 | 25 | x | x | x | | 21 | x | | x | 13 | x | | 5 | x |
| Multi 9-T0 | x | x | x | 10 | | x | x | | x | x | 4 | | 4 | x |
| Multi 11-T0 | x | x | x | 8 | | x | x | | x | x | 4 | | 3 | x |
| Multi 13-T0 | x | x | x | 6 | | x | x | | x | x | x | | x | x |
| Multi 14-T0 | x | x | x | 6 | | x | x | | x | x | x | | x | x |
| Multi 15-T0 | x | x | x | 4 | | x | x | | x | x | 3 | | x | x |

### Example7. Data from Mouse Melanoma Cell Line

**Table 5** presents normalized IC50 (nM) data (K1735 mouse melanoma cell line) for various constructs represented by the structure below. See, Sipes, et al., J. Bilogical Chem 1999, 274, 2275-22762 for experimental procedure.

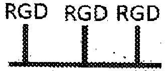

**Table 5. Normalized IC₅₀ (nM) data for K1735 Clone 19 mouse melanoma cell line.**

| K1735 Clone 19 | IC50 (nM) |
|---|---|
| RGD | 741.04 ± 9.02 |
| Three sidechain PNA-Multi 3-T0 | 15.70 ± 1.09 |
| Three sidechain PNA-Multi 5-T0 | 3.60 ± 0.79 |
| Three sidechain PNA-Multi 7-T0 | 4.04 ± 0.96 |
| Three sidechain PNA-Multi 9-T0 | 3.21 ± 0.91 |
| Three sidechain PNA-Multi 11-T0 | 2.53 ± 0.69 |

### Example 8. Incorporation of Quadruplex-Forming Structures into an Quadruplex-Forming Structures for Inhibition of Viral Attachment to Host Cells

MNPs of the instant invention can be utilized for the incorporation of such molecules into quadruplex-forming structures for inhibition of viral attachment to host cells. Quadruplex-forming oligonucleotides have potent activity for inhibiting the attachment of HIV to host cells (the molecule zintevir is one example, see Urata, H. et al. Biochem. Biophys. Res. Commun. 2004, 31, 55). Zintevir is a single-stranded oligonucleotide derivative that adopts a quadruplex structure. PNA is known to form quadruplex structures, and the most stable quadruplexes involve a combination of PNA and DNA. In our invention, MNP becomes part of a quadruplex that inhibits viral entry. Various sidechains of the MNP can be utilized and optimized for the activity needed for a particular viral agent.

### Example 9. Inhibition of Cancer Metastasis In Vivo with an RGD-Conjugated MNP.

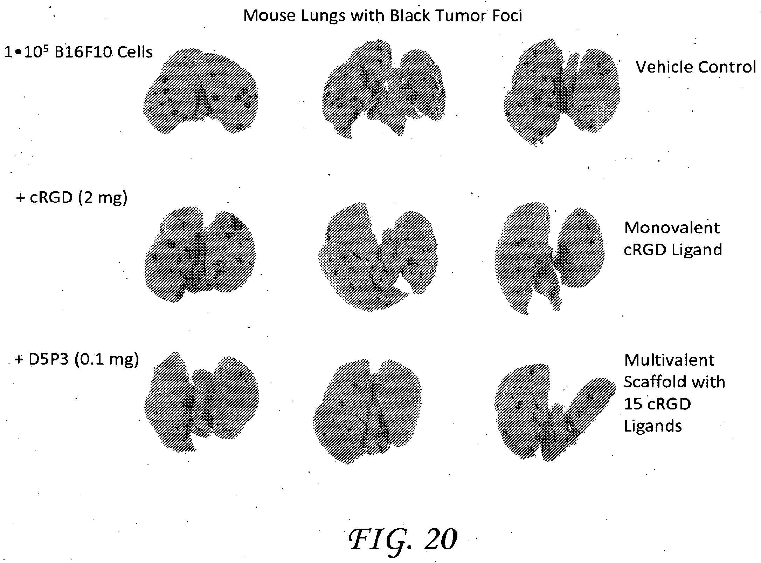

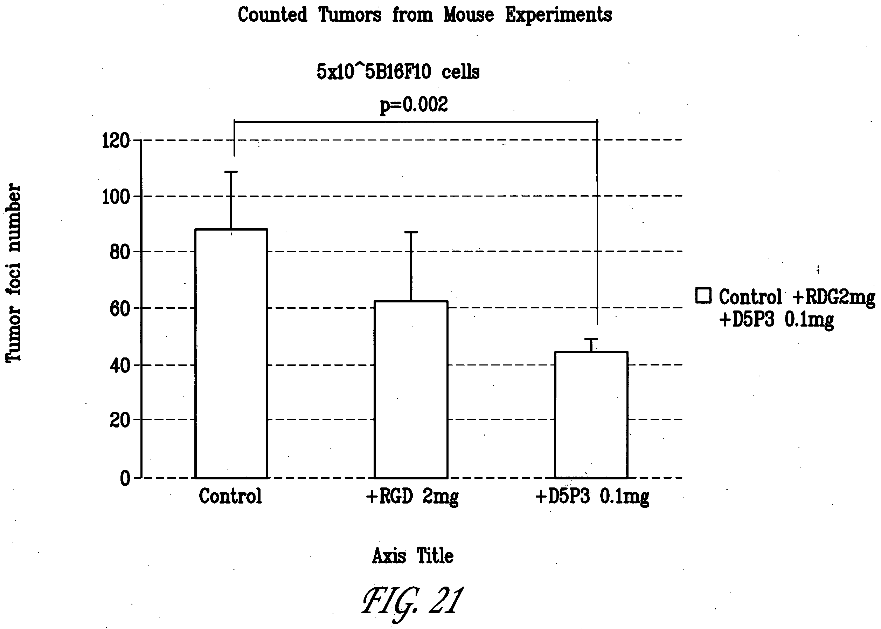

Experiments were conducted to assess the ability of an RGD conjugated PNA to inhibit metastasis of *cancer in vivo.* B16F10 mouse melanoma cells were detached from culture flask and resuspended in serum-free RPMI-1640 medium supplemented with 0.1% BSA at a concentration of 5x10⁶ cells/ml. Cyclo-RGD or RGD-conjugated PNA complexes (MNP D5P3) were then mixed with cells and 0.2 ml aliquots containing 2 mg of cyclo-RGD, 100 µg of MNP D5P3, or PBS (negative control). Suspensions containing 5x10⁵ cells were injected slowly into the lateral tail vein of 8-10 week old female C57BL/6 mice were obtained from Charles River Laboratories. Fourteen days later the animals were euthanized with CO₂ and their lungs were excised and fixed in Bouin's solution (SIGMA). The number of surface melanoma colonies was counted visually (Figure 20). Lung localized tumor foci were significantly reduced in mice injected with MNP D5P3 compared to cyclo-RGD or PBS (Figure 21).

## Claims

1. A composition comprising:
strands of polynucleotide and
strands of PNA, each PNA strand comprising:
(i) from 7 to 50 nucleobase subunits; and
(ii) one or more gamma substituents;
the PNA strands being complementary to at least a portion of at least some of the polynucleotide strands, and
the molar ratio of PNA strands to polynucleotide strands being at least 2:1,
wherein the gamma substituent is capable of binding to a protein.

2. The composition of claim 1, wherein the PNA strand comprises 7, 8, 9, 10, 11, 12, 13, 14, 15, 16, 17, 18, 19, or 20 nucleobase subunits.

3. The composition of any one of the previous claims, wherein each PNA strand comprises 1, 2, or 3 gamma substituents.

4. The composition of claim 1, wherein the PNA comprises a backbone having at least one cyclopentyl residue.

5. The composition of any one of claims 1-4, wherein the protein is a transmembrane protein, lipid-anchored protein, or peripheral protein.

6. The composition of any one of claims 1-4, wherein the protein is a cellular receptor or adhesion molecule.

7. The composition of any one of claims 1-4, wherein the protein is an integrin, cadherin, selectin, addressin, G protein-coupled receptor, or toll-like receptor.

8. The composition of any one of claims 1-7, wherein the molar ratio of PNA strands to polynucleotide strands is 3:1 to 7:1, optionally wherein:
(i) the molar ratio of PNA strands to polynucleotide strands is 3:1, 4:1, 5:1, 6:1 or 7:1; or
(ii) the molar ratio of PNA strands to polynucleotide strands is 4:1 to 6:1.

9. The composition of claim 1 or 3, wherein the gamma substituents, independently, are
-RNX¹X²,
where:
R is a C₁-C₁₂ alkyl,
X¹ and X² are, independently, H, biomolecules, metal ligands, Michael acceptors, azides, alkynes, or thiols;
wherein at least one of X¹ and X² is other than H.

10. The composition of claim 9, wherein X¹ and X² are, independently, H, polypeptide, mannose, lactose, nucleic acid derivatives, oligonucleotides, RGD (Arg-Gly- Asp) or cyclic RGD.

11. The composition of claim 9, wherein X¹ and X² are, independently, H, cyclodextrins, porphyrins, polyhedral cage compounds containing boron, DOTA, DTPA, a crown ether, a cryptand, a pyridine-containing ligand, or calixarenes.

12. A composition of any one of claims 1 to 4, wherein the composition is detectably labeled, for use in a method of detecting the presence of a cellular surface protein in a subject.

13. The composition for use according to claim 12, wherein the detectable label is a fluorescent label, radio label, biotin, DOTA, DTPA, or a radionuclide.

## Patentansprüche

1. Zusammensetzung, umfassend:
Stränge von Polynukleotid und
Stränge von PNA, wobei jeder PNA-Strang Folgendes umfasst:
(i) 7 bis 50 Nukleobase-Untereinheiten; und
(ii) einen oder mehrere Gamma-Substituenten;
wobei die PNA-Stränge zu wenigstens einem Teil wenigstens einiger der Polynukleotidstränge komplementär sind und
das Molverhältnis von PNA-Strängen zu Polynukleotidsträngen wenigstens 2:1 beträgt, wobei der Gamma-Substituent zur Bindung an ein Protein fähig ist.

2. Zusammensetzung nach Anspruch 1, wobei der PNA-Strang 7, 8, 9, 10, 11, 12, 13, 14, 15, 16, 17, 18, 19 oder 20 Nukleobase-Untereinheiten umfasst.

3. Zusammensetzung nach einem der vorhergehenden Ansprüche, wobei jeder PNA-Strang 1, 2 oder 3 Gamma-Substituenten umfasst.

4. Zusammensetzung nach Anspruch 1, wobei die PNA ein Rückgrat mit wenigstens einem Cyclopentylrest umfasst.

5. Zusammensetzung nach einem der Ansprüche 1-4, wobei es sich bei dem Protein um ein Transmembran-protein, lipid-verankertes Protein oder peripheres Protein handelt.

6. Zusammensetzung nach einem der Ansprüche 1-4, wobei es sich bei dem Protein um einen zellulären Rezeptor oder ein zelluläres Adhäsionsmolekül handelt.

7. Zusammensetzung nach einem der Ansprüche 1-4, wobei es sich bei dem Protein um ein Integrin, Cadherin, Selectin, Addressin, einen G-proteingekoppelten Rezeptor oder Toll-like-Rezeptor handelt.

8. Zusammensetzung nach einem der Ansprüche 1-7, wobei das Molverhältnis von PNA-Strängen zu Polynukleotidsträngen 3:1 bis 7:1 beträgt, gegebenenfalls wobei:
(i) das Molverhältnis von PNA-Strängen zu Polynukleotidsträngen 3:1, 4:1, 5:1, 6:1 oder 7:1 beträgt; oder
(ii) das Molverhältnis von PNA-Strängen zu Polynukleotidsträngen 4:1 bis 6:1 beträgt.

9. Zusammensetzung nach Anspruch 1 oder 3, wobei es sich bei den Gamma-Substituenten unabhängig um
-RNX¹X²
handelt, wo:
R für ein C₁-C₁₂-Alkyl steht,
X¹ und X² unabhängig für H, Biomoleküle, Metallliganden, Michael-Akzeptoren, Azide, Alkine oder Thiole stehen;
wobei wenigstens einer X¹ und X² von H verschieden ist.

10. Zusammensetzung nach Anspruch 9, wobei X¹ und X² unabhängig für H, Polypeptid, Mannose, Lactose, Nukleinsäurederivate, Oligonukleotide, RGD (Arg-Gly-Asp) oder cyclisches RGD stehen.

11. Zusammensetzung nach Anspruch 9, wobei X¹ und X² unabhängig für H, Cyclodextrine, Porphyrine, polyhedrale Cage-Verbindungen mit Bor, DOTA, DTPA, einen Kronenether, einen Cryptanden, einen pyridinhaltigen Liganden oder Calixarene stehen.

12. Zusammensetzung nach einem der Ansprüche 1 bis 4, wobei die Zusammensetzung nachweisbar markiert ist, zur Verwendung bei einem Verfahren zum Nachweis des Vorliegens eines Zelloberflächenproteins bei einem Individuum.

13. Zusammensetzung zur Verwendung gemäß Anspruch 12, wobei es sich bei der nachweisbaren Markierung um eine Fluoreszenzmarkierung, radioaktive Markierung, Biotin, DOTA, DTPA oder ein Radionuklid handelt.

## Revendications

1. Composition comprenant :
des brins d'un polynucléotide et
des brins de PNA, chaque brin de PNA comprenant :
(i) de 7 à 50 sous-unités de nucléobases ; et
(ii) un ou plusieurs substituant(s) en gamma ;
les brins de PNA étant complémentaires d'au moins une partie d'au moins quelques-uns des brins d'un polynucléotide, et
le rapport molaire des brins de PNA aux brins d'un polynucléotide étant d'au moins 2:1,
dans lequel le substituant en gamma est capable de se lier à une protéine.

2. Composition selon la revendication 1, dans laquelle le brin de PNA comprend 7, 8, 9, 10, 11, 12, 13, 14, 15, 16, 17, 18, 19 ou 20 sous-unités de nucléobases.

3. Composition selon l'une quelconque des revendications précédentes, dans laquelle chaque brin de PNA comprend 1, 2 ou 3 substituant(s) en gamma.

4. Composition selon la revendication 1, dans laquelle le PNA comprend un squelette ayant au moins un résidu de cyclopentyle.

5. Composition selon l'une quelconque des revendications 1 à 4, dans laquelle la protéine est une protéine transmembranaire, une protéine ancrée à un lipide ou une protéine périphérique.

6. Composition selon l'une quelconque des revendications 1 à 4, dans laquelle la protéine est un récepteur cellulaire ou une molécule d'adhérence.

7. Composition selon l'une quelconque des revendications 1 à 4, dans laquelle la protéine est une intégrine, une cadhérine, une sélectine, une addressine, un récepteur couplé à la protéine G ou un récepteur du type toll.

8. Composition selon l'une quelconque des revendications 1 à 7, dans laquelle le rapport molaire des brins de PNA aux brins d'un polynucléotide est de 3:1 à 7:1, facultativement dans laquelle :
(i) le rapport molaire des brins de PNA aux brins d'un polynucléotide est de 3:1, 4:1, 5:1, 6:1 ou 7:1 ; ou
(ii) le rapport molaire des brins de PNA aux brins d'un polynucléotide est de 4:1 à 6:1.

9. Composition selon la revendication 1 ou la 3, dans laquelle les substituants en gamma sont indépendamment,
- RNX¹X²,
où :
R est un alkyle en C₁-C₁₂,
X¹ et X² sont indépendamment H, des biomolécules, des ligands métalliques, des accepteurs de Michael, des azides, des alcynes ou des thiols ;
dans laquelle au moins un parmi les X¹ et X² est autre que H.

10. Composition selon la revendication 9, dans laquelle X¹ et X² sont indépendamment H, un polypeptide, le mannose, le lactose, des dérivés d'acides nucléiques, des oligonucléotides, un RGD (Arg-Gly-Asp) ou un RGD cyclique.

11. Composition selon la revendication 9, dans laquelle X¹ et X² sont indépendamment H, des cyclodextrines, des porphyrines, des composés à cage polyédrique contenant du bore, le DOTA, le DTPA, un éther couronne, un cryptant, un ligand contenant de la pyridine ou des calixarènes.

12. Composition selon l'une quelconque des revendications 1 à 4, dans laquelle la composition est marquée de manière détectable, pour être utilisée dans un procédé de détection de la présence d'une protéine de surface cellulaire chez un sujet.

13. Composition destinée à être utilisée selon la revendication 12, dans laquelle le marqueur détectable est un marqueur fluorescent, un radio-marqueur, la biotine, le DOTA, le DTPA ou un radionucléide.
